# EUROPEAN PATENT APPLICATION

(11) **EP 4 276 115 A1**
(43) Date of publication of application: **15.11.2023**
(21) Application number: 22736573.1
(22) Date of filing: 07.01.2022
(51) Int. Cl.: C07K 16/46, C12N 15/13, C12N 15/53, C12N 5/00, A61K 39/395, A61P 35/00

(54) **ANTI-PD-1/ANTI-CD47 NATURAL ANTIBODY STRUCTURE-LIKE HETERODIMERIC FORM BISPECIFIC ANTIBODY AND PREPARATION THEREOF**

(30) Priority: 08.01.2021 CN 202110025240
(71) Applicant: Beijing Hanmi Pharmaceutical Co., Ltd., Beijing 101312 (CN)
(72) Inventor: LIU, Jiawang, Beijing 101312 (CN); YANG, Yaping, Beijing 101312 (CN); ZHAO, Siqi, Beijing 101312 (CN); LIU, Yang, Beijing 101312 (CN); SONG, Nanmeng, Beijing 101312 (CN); ZHANG, Hongjuan, Beijing 101312 (CN); YANG, Dongxue, Beijing 101312 (CN); ZHANG, Lanxin, Beijing 101312 (CN); WANG, Jing, Beijing 101312 (CN); XU, Jiangcheng, Beijing 101312 (CN); LEE, Kyoung Woo, Beijing 101312 (CN)
(74) Representative: Høiberg P/S
(86) International application number: PCT/CN2022/070625
(87) International publication number: WO 2022/148411

(57) **Abstract**

The present invention provides an anti-PD-l/anti-CD47 bispecific antibody and a preparation method therefor. The antibody has characteristics of a natural IgG, and is a highly stable heterodimeric form without heavy and light chain mismatching. The bispecific antibody can bind two target molecules at the same time and has a smaller side effect.

## Description

### Field of the Invention

The present invention relates to an anti-PD-L1/anti-CD47 bispecific antibody in the form of a natural antibody structure-like heterodimer and preparation thereof. In particular, the present invention provides an anti-PD-L1/anti-CD47 bispecific antibody in the form of a highly stable heterodimer with the characteristics of natural IgGs and no heavy and light chain mismatches, as well as the preparation method thereof.

### Background of the Invention

Programmed death ligand-1 (PD-L1) is a ligand for immune checkpoint programmed death receptor-1 (programmed death-1, PD-1). PD-L1 belongs to the B7 family and is expressed in an inducible manner on the surface of a variety of immune cells, including T cells, B cells, monocytes, macrophages, DC cells, endothelial cells, epidermal cells, etc. After binding to PD-1, PD-L1 is mainly involved in the negative regulation of T cell activation, and is able to regulate the strength and duration of an immune response. In addition to acting as a ligand for PD-1, PD-L1 can also act as a ligand for CD80, to deliver negative regulatory signals to T cells and to induce T cell immune tolerance (Autoimmun Rev, 2013, 12(11): 1091-1100.; Front Immunol, 2013, 4: 481.; Nat Rev Cancer, 2012, 12(4): 252-264.; Trends Mol Med. 2015 Jan;21(1):24-33.; Clin Cancer Res. 2012 Dec 15;18(24):6580-7.). Normally, PD-L1 and PD-1 can mediate and maintain body's autoimmune tolerance, prevent the excessive activation of immune system from damaging its own tissues during an inflammatory reaction, and thus play a positive role in preventing the occurrence of an autoimmune disease. Under pathological conditions, PD-L1 and PD-1 are involved in tumor immunity as well as the occurrence and development of various autoimmune diseases. Several studies have reported that PD-L1 is highly expressed in a variety of tumor tissues, PD-1 is highly expressed in tumor infiltrating lymphocytes, and the overexpressions of PD-L1 and PD-1 are tightly associated with poor clinical prognosis of tumors (Anticancer Agents Med Chem. 2015; 15(3):307-13.; Hematol Oncol Stem Cell Ther. 2014 Mar;7(1):1-17.; Trends Mol Med. 2015 Jan;21(1):24-33.; Immunity. 2013 Jul 25;39(1):61-73. J Clin Oncol. 2015 Jun 10;33(17):1974-82.). Blocking the interactions of PD-L1/PD-1 and CD80/PD-L1 with a PD-L1 monoclonal antibody has shown good anti-tumor effects in both preclinical experimental studies and in clinical trials. Currently, anti-PD-L1 monoclonal antibody has been approved for the treatment of various tumors such as non-small cell lung cancer and urothelial carcinoma. However, only a small proportion of tumor patients can benefit from this type of monoclonal antibody therapy, and most patients do not respond to this type of monoclonal antibody therapy at all (Expert Opin Ther Targets. 2014 Dec;18(12):1407-20. Oncology (Williston Park). 2014 Nov;28 Suppl 3:15-28.).

CD47 (also known as integrin-associated protein), is a transmembrane protein with a molecular weight of 50 Kd of the immunoglobulin superfamily. CD47 is widely expressed on a variety of cells, but its expression on a variety of tumor cells is significantly enhanced (Proc Natl Acad Sci USA, 2012, 109(17): 6662-6667). The ligand of CD47 is signal-regulatory protein α (SIRPα), which is mainly expressed on macrophages. After binding to CD47, it transmits a signal of "don't eat me" and inhibits the phagocytosis of macrophages (Curr Opin Immunol, 2009, 21(1):47-52). Anti-CD47 antibodies can block the CD47-SIRPα signaling pathway, thereby playing an anti-tumor role. At present, a variety of anti-CD47 monoclonal antibodies have entered the clinical research stage for the treatment of various blood tumors and solid tumors. However, since CD47 is also expressed on the surface of red blood cells and the like, these anti-CD47 treatments may cause adverse reactions such as severe anemia and thrombocytopenia, and their bioavailability is low.

Therefore, there is still a need in the present field to develop a novel therapeutic drug that binds to the PD-L1 and CD47 simultaneously.

### Summary of the Invention

The present invention provides a novel bifunctional antibody in the form of a highly stable heterodimer with the characteristics of natural IgGs and no heavy and light chain mismatches and capable of binding to PD-L1 and CD47 simultaneously and the preparation method thereof. The bifunctional antibody tends to bind to the tumor cells highly expressing PD-L1 and the T cell expressing CD47 simultaneously, and thus exerts efficient and specific killing effects with lower toxicity and side effects.

One aspect of the present invention relates to a bispecific antibody comprising a first antigen-binding functional region that specifically binds to PD-L1 and a second antigen-binding functional region that specifically binds to CD47, wherein the first antigen-binding functional region that specifically binds PD-L1 comprises:
(A) a heavy chain variable region, comprising
   (a) HCDR1, which comprises the amino acid sequence set forth in SEQ ID NO: 37,
   (b) HCDR2, which comprises the amino acid sequence set forth in SEQ ID NO: 38, and
   (c) HCDR3, which comprises the amino acid sequence set forth in SEQ ID NO: 39; and
(B) a light chain variable region, comprising
   (a) LCDR1, which comprises the amino acid sequence set forth in SEQ ID NO: 40,
   (b) LCDR2, which comprises the amino acid sequence set forth in SEQ ID NO: 41, and
   (c) LCDR3, which comprises the amino acid sequence set forth in SEQ ID NO: 42.

In some embodiments, the second antigen-binding functional region of the bispecific antibody that specifically binds to CD47 comprises
(A) a heavy chain variable region, comprising
   (a) HCDR1, which comprises the amino acid sequence set forth in SEQ ID NO: 43,
   (b) HCDR2, which comprises the amino acid sequence set forth in SEQ ID NO: 44, and
   (c) HCDR3, which comprises the amino acid sequence set forth in SEQ ID NO: 45; and
(B) a light chain variable region, comprising
   (a) LCDR1, which comprises the amino acid sequence set forth in SEQ ID NO: 46,
   (b) LCDR2, which comprises the amino acid sequence set forth in SEQ ID NO: 47, and
   (c) LCDR3, which comprises the amino acid sequence set forth in SEQ ID NO: 48.

In some embodiments, the first antigen-binding functional region of the bispecific antibody that specifically binds to PD-L1 comprises
(A) a heavy chain variable region, comprising
   (a) HCDR1, which comprises the amino acid sequence set forth in SEQ ID NO: 37,
   (b) HCDR2, which comprises the amino acid sequence set forth in SEQ ID NO: 38, and
   (c) HCDR3, which comprises the amino acid sequence set forth in SEQ ID NO: 39; and
(B) a light chain variable region, comprising
   (a) LCDR1, which comprises the amino acid sequence set forth in SEQ ID NO: 40,
   (b) LCDR2, which comprises the amino acid sequence set forth in SEQ ID NO: 41, and
   (c) LCDR3, which comprises the amino acid sequence set forth in SEQ ID NO: 42; and
the second antigen-binding functional region that specifically binds to CD47 comprises
(A) a heavy chain variable region, comprising
   (a) HCDR1, which comprises the amino acid sequence set forth in SEQ ID NO: 43,
   (b) HCDR2, which comprises the amino acid sequence set forth in SEQ ID NO: 44, and
   (c) HCDR3, which comprises the amino acid sequence set forth in SEQ ID NO: 45; and
(B) a light chain variable region, comprising
   (a) LCDR1, which comprises the amino acid sequence set forth in SEQ ID NO: 46,
   (b) LCDR2, which comprises the amino acid sequence set forth in SEQ ID NO: 47, and
   (c) LCDR3, which comprises the amino acid sequence set forth in SEQ ID NO: 48.

The six CDRs of the first antigen-binding functional region that specifically binds to PD-L1 as set forth in SEQ ID Nos. 37-42, and the six CDRs of the second antigen-binding functional region that specifically binds to CD47 as set forth in SEQ ID Nos. 43-48 are the CDRs of the heavy and light chain variable regions of the monoclonal antibodies obtained by the inventors using hybridoma technology with human PD-L1 and CD47 as the antigens. Said monoclonal antibodies are different from the anti-PD-L1 antibodies and anti-CD47 antibodies known in the prior art and have higher biological activities, such as higher binding specificity, anti-tumor activity and so on.

In some embodiments, the six CDR sequences of the first antigen-binding functional region that specifically binds to PD-L1 may have at least 70%, such as at least 75%, 80%, 85%, 90%, 95% or higher identity to the sequences set forth in SEQ ID Nos. 37-42, respectively, and retain the biological activities of the corresponding parent sequences; or may be variant sequences containing one or more, such as 1, 2, 3 or more amino acid deletions, substitutions and/or additions compared to the sequences set forth in SEQ ID Nos. 37-42, respectively, and retaining the biological activities of the corresponding parent sequences.

In some embodiments, the six CDR sequences of the second antigen-binding functional region that specifically binds to CD47 may have at least 70%, such as at least 75%, 80%, 85%, 90%, 95% or higher identity to the sequences set forth in SEQ ID Nos. 43-48, respectively, and retain the biological activities of the corresponding parent sequences; or may be variant sequences containing one or more, such as 1, 2, 3 or more amino acid deletions, substitutions and/or additions compared to the sequences set forth in SEQ ID Nos. 43-48, respectively, and retaining the biological activities of the corresponding parent sequences.

In some embodiments, the first antigen-binding functional region that specifically binds to PD-L1 comprises a heavy chain variable region and a light chain variable region, wherein the heavy chain variable region comprises the amino acid sequence set forth in SEQ ID NO: 6; or has at least 70%, such as at least 75%, 80%, 85%, 90%, 95%, 96%, 97%, 98%, 99% or higher identity to the sequence set forth in SEQ ID NO: 6 and retains the biological activities of the corresponding parent sequence; or contains one or more, such as 1, 2, 3, 4, 5, 6, 7, 8, 9, 10, 15, 20 or more amino acid deletions, substitutions and/or additions compared to the sequence set forth in SEQ ID NO: 6 and retains the biological activities of the corresponding parent sequence; and the light chain variable region comprises the amino acid sequence set forth in SEQ ID NO: 2; or has at least 70%, such as at least 75%, 80%, 85%, 90%, 95%, 96%, 97%, 98%, 99% or higher identity to the sequence set forth in SEQ ID NO: 2 and retains the biological activities of the corresponding parent sequence; or contains one or more, such as 1, 2, 3, 4, 5, 6, 7, 8, 9, 10, 15, 20 or more amino acid deletions, substitutions and/or additions compared to the sequence set forth in SEQ ID NO: 2 and retains the biological activities of the corresponding parent sequence.

In some embodiments, the second antigen-binding functional region that specifically binds to CD47 comprises a heavy chain variable region and a light chain variable region, wherein the heavy chain variable region comprises the amino acid sequence set forth in SEQ ID Nos: 14, 20, 24, 28, 32 or 36; or has at least 70%, such as at least 75%, 80%, 85%, 90%, 95%, 96%, 97%, 98%, 99% or higher identity to the sequence set forth in SEQ ID Nos: 14, 20, 24, 28, 32 or 36 and retains the biological activities of the corresponding parent sequence; or may be a variant sequence containing one or more, such as 1, 2, 3, 4, 5, 6, 7, 8, 9, 10, 15, 20 or more amino acid deletions, substitutions and/or additions compared to the sequence set forth in SEQ ID NO: 6 and retaining the biological activities of the corresponding parent sequence; and the light chain variable region comprises the amino acid sequence set forth in SEQ ID Nos: 12, 18, 22, 26, 30 or 34; or has at least 70%, such as at least 75%, 80%, 85%, 90%, 95%, 96%, 97%, 98%, 99% or higher identity to the sequence set forth in SEQ ID Nos: 12, 18, 22, 26, 30 or 34 and retains the biological activities of the corresponding parent sequence; or may be a variant sequence containing one or more, such as 1, 2, 3, 4, 5, 6, 7, 8, 9, 10, 15, 20 or more amino acid deletions, substitutions and/or additions compared to the sequence set forth in SEQ ID NO: 6 and retaining the biological activities of the corresponding parent sequence.

In some embodiments, the first antigen-binding functional region that specifically binds to PD-L1 comprises a heavy chain variable region and a light chain variable region, wherein the heavy chain variable region comprises the amino acid sequence set forth in SEQ ID NO: 6, and the light chain variable region comprises the amino acid sequence set forth in SEQ ID NO: 2; and the second antigen-binding functional region that specifically binds to CD47 comprises a heavy chain variable region and a light chain variable region selected from the following combinations:
a) the heavy chain variable region comprising the amino acid sequence set forth in SEQ ID NO: 14, and the light chain variable region comprising the amino acid sequence set forth in SEQ ID NO: 12;
b) the heavy chain variable region comprising the amino acid sequence set forth in SEQ ID NO: 20, and the light chain variable region comprising the amino acid sequence set forth in SEQ ID NO: 18;
c) the heavy chain variable region comprising the amino acid sequence set forth in SEQ ID NO: 24, and the light chain variable region comprising the amino acid sequence set forth in SEQ ID NO: 22;
d) the heavy chain variable region comprising the amino acid sequence set forth in SEQ ID NO: 28, and the light chain variable region comprising the amino acid sequence set forth in SEQ ID NO: 26;
e) the heavy chain variable region comprising the amino acid sequence set forth in SEQ ID NO: 32, and the light chain variable region comprising the amino acid sequence set forth in SEQ ID NO: 30;
f) the heavy chain variable region comprising the amino acid sequence set forth in SEQ ID NO: 36, and the light chain variable region comprising the amino acid sequence set forth in SEQ ID NO: 34.

In some embodiments, the first antigen-binding functional region and the second antigen-binding functional region of the bispecific antibody are selected from the group consisting of Fab fragments, scFv fragments, and variable domain fragments Fv.

In further embodiments, the first antigen-binding functional region and the second antigen-binding functional region of the bispecific antibody are both Fab fragments.

In some embodiments, the Fab fragment of the bispecific antibody comprises a different first heavy chain variable region and a different second heavy chain variable region, as well as a different first light chain variable region and a different second light chain variable region.

In some embodiments, one of the first antigen-binding functional region and the second antigen-binding functional region of the bispecific antibody is a Fab fragment, and the other is a scFv.

In some embodiments, the bispecific antibody comprises a first Fc chain and a second Fc chain,
wherein the first Fc chain and the second Fc chain are both immunoglobulin G Fc fragments containing amino acid substitutions, and the first Fc chain and the second Fc chain together constitute a heterodimer that can bind to the Fc receptor;
wherein the first Fc chain and the second Fc chain are linked to the first antigen-binding functional region and the second antigen-binding functional region, respectively, via a covalent bond or a linker; and
wherein either the first Fc chain or the second Fc chain comprises amino acid substitutions T366L and D399R at positions 366 and 399, and the other comprises amino acid substitutions L351E, Y407L and K409V at positions 351, 407 and 409, wherein the amino acid positions are numbered according to the Kabat EU index numbering system.

Herein, unless otherwise specified, the amino acid positions of an antibody are numbered according to the Kabat EU index numbering system.

In some embodiments, the weight ratios of the homodimers formed by the first Fc chain and the first antigen-binding functional region covalently linked thereto and by the second Fc chain and the second antigen -binding functional region covalently linked thereto of said bispecific antibody are less than 50%, such as less than 45%, 40%, 35%, 30%, 25%, 20% or less of the total amount of all the polypeptide chains, in a reducing agent-containing solution in which no other polypeptides present except for said Fc chains and the antigen-binding functional regions hereinabove.

In some embodiments, the first antigen-binding functional region of the bispecific antibody comprises the amino acid sequences set forth in SEQ ID Nos: 2 and 6.

In some embodiments, the second antigen-binding functional region of the bispecific antibody comprises the amino acid sequences set forth in SEQ ID Nos: 12, 14, 18, 20, 22, 24, 26, 28, 30, 32, 34 and 36. Those skilled in the art would know that the combinations of the amino acid sequences should follow the biological laws, i.e., light and heavy chains or the variable regions, the antigen-binding fragments thereof, such as SEQ ID NO: 12 and SEQ ID NO: 14, and SEQ ID NO: 18 and SEQ ID NO: 14, should match each other.

In some embodiments, both the first and second light chains of the bispecific antibody comprise the amino acid sequence set forth in SEQ ID No: 4.

In some embodiments, one of the first and second heavy chains of the bispecific antibody comprises the amino acid sequence set forth in SEQ ID No: 8 or 10, and the other comprises the amino acid sequence set forth in SEQ ID No: 16.

In some embodiments, the bispecific antibody comprises a first heavy chain and first light chain pair that specifically binds to PD-L1, wherein the first heavy chain has an heavy chain variable region comprising the amino acid sequence set forth in SEQ ID NO: 6, and a heavy chain constant region comprising the amino acid sequence set forth in SEQ ID NO: 8 or 10; the first light chain has a light chain variable region comprising the amino acid sequence set forth in SEQ ID NO: 2, and a light chain constant region comprising the amino acid sequence set forth in SEQ ID NO: 4.

In some embodiments, the bispecific antibody comprises a second heavy chain and second light chain pair that specifically binds to CD47, wherein the second heavy chain has an heavy chain variable region comprising the amino acid sequence set forth in SEQ ID Nos: 14, 20, 24, 28, 32 or 36, and a heavy chain constant region comprising the amino acid sequence set forth in SEQ ID NO: 16; the second light chain has a light chain variable region comprising the amino acid sequence set forth in SEQ ID Nos: 12, 18, 22, 26, 30 or 34, and a light chain constant region comprising the amino acid sequence set forth in SEQ ID NO: 4.

A second aspect of the present invention relates to an isolated polynucleotide encoding the bispecific antibody as described above.

In some embodiments, the nucleotide sequences encoding the amino acid sequences of the first antigen-binding functional region comprise the nucleotide sequences set forth in SEQ ID Nos: 1 and 5.

In some embodiments, the nucleotide sequences encoding the amino acid sequences of the second antigen-binding functional region comprise the nucleotide sequences set forth in SEQ ID Nos: 11, 13, 17, 19, 21, 23, 25, 27, 29, 31, 33 and 35. As described above, the combinations of the nucleotide sequences should follow the biological laws, i.e., the nucleotide sequences encoding light and heavy chains or the variable regions, the antigen-binding fragments thereof, such as SEQ ID NO: 11 and SEQ ID NO: 13, and SEQ ID NO: 17 and SEQ ID NO: 13, should match each other.

In some embodiments, both the nucleotide sequence encoding the amino acid sequences of the first light chain and the second light chain both comprises the nucleotide sequence set forth in SEQ ID NO: 3.

In some embodiments, the nucleotide sequence encoding the amino acid sequence of either the first heavy chain or the second heavy chain comprises the nucleotide sequence set forth in SEQ ID NO: 7 or 9, and the nucleotide sequence encoding the amino acid sequence of the other comprises the nucleotide sequence set forth in SEQ ID NO: 15.

A third aspect of the present invention relates to a recombinant expression vector comprising the isolated polynucleotide as described above.

In some embodiments, the expression vector is the plasmid vector XOGC modified from the pCDNA vector.

A fourth aspect of the present invention relates to a host cell comprising the isolated polynucleotide as described above or the recombinant expression vector as described above.

In some embodiments, the host cell is selected from the group consisting of human embryonic kidney cells HEK293, or HEK293T, HEK293E, HEK293F that are modified from HEK293 cells; hamster ovary cells CHO, or CHO-S, CHO-dhfr⁻, CHO/DG44, ExpiCHO that are modified from CHO cells; *Escherichia coli,* or *E. coli* BL21, BL21(DE3), Rosetta, Origami that are modified from *E. coli;* Yeast, or *Pichia, Saccharomyces cerevisiae, Kluyveromyces lactis, Hansenula polymorpha* that are modified from Yeast; insect cells, or High5, SF9 cells that are modified from insect cells; plant cells; mammalian mammary gland cells; somatic cells.

A fifth aspect of the present invention relates to a composition comprising the bispecific antibody as described above or the isolated polynucleotide as described above or the recombinant expression vector as described above or the host cell as described above, and a pharmaceutically acceptable carrier.

A sixth aspect of the present invention relates to a method of producing the bispecific antibody as described above, including the steps of:
1) Expressing the isolated polynucleotide as described above or the recombinant expression vector as described above in host cells, respectively;
2) Reducing the proteins respectively expressed in the host cells; and
3) Mixing the reduced proteins, and then oxidizing the mixture.

In some embodiments, the reduction step includes 1) performing the reduction reaction in the presence of a reducing agent selected from the group consisting of 2-mercaptoethylamine, dithiothreitol, tris(2-carboxyethyl) phosphine or other chemical derivatives thereof; 2) Removing the reducing agent. In some embodiments, the reducing agent is dithiothreitol at a concentration of 0.1 mM or higher, such as 0.1 mM, 0.2 mM, 0.3 mM, 0.4 mM, 0.5 mM or higher, and the reaction is performed at 4°C for at least 3 hours, such as 3.5 hours, 4 hours, 4.5 hours, 5 hours, 5.5 hours, 6 hours or longer.

In some embodiments, the oxidation step is conducted in the air or in the presence of an oxidizing agent selected from the group consisting of L-dehydroascorbic acid or chemical derivatives thereof. In some embodiments, the oxidizing agent is L-dehydroascorbic acid at a concentration of 0.5 mM or higher, such as 0.6 mM, 0.7 mM, 0.8 mM, 0.9 mM, 1.0 mM, 1.2 mM, 1.5 mM or higher, and the reaction is performed at 4°C for at least 5 hours, such as 5 hours, 6 hours, 7 hours, 8 hours, 9 hours, or longer.

In some embodiments, the method further includes a step of separation and purification. In some embodiments, the separation and purification include cation resin exchange, anion resin exchange, reverse phase chromatography, affinity chromatography, size exclusion chromatography, and combinations thereof.

A seventh aspect of the present invention relates to use of the bispecific antibody as described above and/or the isolated polynucleotide as described above and/or the recombinant expression vector as described above and/or the host cell as described above and/or the composition as described above, in the manufacture of a medicament for preventing and/or treating diseases in a subject.

An eighth aspect of the present invention relates to the bispecific antibody as described above and/or the isolated polynucleotide as described above and/or the recombinant expression vector as described above and/or the host cell as described above and/or the composition as described above, for use as a medicament for preventing and/or treating diseases in a subject.

A ninth aspect of the present invention relates to a method of preventing and/or treating diseases, including administering to a subject in need thereof the bispecific antibody as described above and/or the isolated bispecific antibody as described above and/or the recombinant expression vector as described above and/or the host cell as described above and/or the composition as described above.

In some embodiments, the subject is a mammal, preferably a human subject.

In some embodiments, the disease is selected from the group consisting of the following tumors: leukemia, lymphoma, myeloma, brain tumor, head and neck squamous cell cancer, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell cancer, melanoma, small cell lung cancer and bone cancer, preferably, the disease is selected from the group consisting of leukemia, lymphoma, and myeloma.

In other words, the present invention designs a novel anti-PD-L1/anti-CD47 bispecific antibody in the form of a natural antibody structure-like heterodimer with the characteristics of natural IgGs and no heavy and light chain mismatches, and thus it is a highly stable anti-PD-L1/anti-CD47 bispecific antibody in a heterodimeric form. The bispecific antibody prepared in the invention is able to bind to two target molecules, PD-L1 and CD47 simultaneously, and to exert better efficacy with fewer side effects than a monotherapeutic agent when applied to treat complicated diseases. Moreover, compared to a combination therapy of multiple medicaments, the bispecific antibody, as a single therapeutic molecule, not only facilitates the use by patients and medical workers, but also simplifies the complex development processes for new drugs.

### Brief description of the figures

Fig. 1 illustrates a chromatogram of elution peaks of the anti-PD-L1 expression product.
Fig. 2 illustrates a chromatogram of elution peaks of the anti-CD47 expression product.
Fig. 3 illustrates a structure of the anti-PD-L1/anti-CD47 heterodimeric antibody molecule.
Fig. 4 illustrates a molecular structure of a half-antibody comprising one heavy chain and one light chain.
Fig. 5 illustrates results of SEC-HPLC analysis of the half-antibody molecule containing one heavy chain and one light chain, wherein panels A and B illustrate the results of the anti-PD-L1 half antibody molecule and the anti-CD47 half antibody molecule, respectively.
Fig. 6 illustrates results of SEC-HPLC analysis of the anti-PD-L1/anti-CD47 heterodimeric antibody molecule.
Fig. 7 illustrates results of CE analysis of the anti-PD-L1/anti-CD47 heterodimeric antibody molecule.
Fig. 8. Panel A illustrates the affinities of several anti-PD-L1/anti-CD47 heterodimeric antibodies to PD-L1, panel B illustrates the affinities of several anti-PD-L1/anti-CD47 heterodimeric antibody to CD47.
Fig. 9. Panel A illustrates the activities of several anti-PD-L1/anti-CD47 heterodimeric antibody blocking the binding to PD-L1/PD-1, panel B illustrates the activities of several anti-PD-L1/anti-CD47 heterodimeric antibody blocking the binding to PD-L1/CD80, and panel C illustrates the activities of several anti-PD-L1/anti-CD47 heterodimeric antibody blocking the binding to CD47/SIRPα.
Fig. 10. Panel A illustrates the binding activity of the anti-CD47 monoclonal antibody to HCC827 cells and RBCs, panels B and C show the binding activity of the anti-PD-L1/anti-CD47 heterodimeric antibody to HCC827 cells and RBCs.
Fig. 11 illustrates the T cell regulatory activity of the anti-PD-L1/anti-CD47 heterodimeric antibody.
Fig. 12 illustrates the phagocytic activity of macrophages on tumor cells mediated by the anti-PD-L1/anti-CD47 heterodimeric antibody.
Fig. 13 illustrates the anti-tumor efficacy of the anti-PD-L1/anti-CD47 heterodimeric antibody in the xenograft tumor model.
Fig. 14 illustrates the anti-tumor efficacy of the anti-PD-L1/anti-CD47 heterodimeric antibody in the gene knock-in mouse tumor model.
Fig. 15 illustrates that the anti-PD-L1/anti-CD47 heterodimeric antibody BH3012h has a stronger anti-tumor efficacy than the corresponding anti-PD-L1 bivalent monoclonal antibody and the anti-CD 47 bivalent monoclonal antibody.

### Detailed description of the embodiments

### Definitions

As used herein, the term "antibody" is used in its broadest sense and refers to a protein containing antigen-binding site(s), including natural antibodies and artificial antibodies with various structures, including but not limited to intact antibodies and antigen-binding fragments of the antibodies.

As used herein, the term "bispecific antibody" comprises antigen binding domains specifically binding to epitopes on two different biological molecules. Unless otherwise specified, the order of the antigens bound by the bispecific antibody is arbitrary. That is, in some embodiments, the terms "anti-PD-L1/CD47 bispecific antibody" and "anti-CD47/PD-L1 bispecific antibody" are used interchangeably. In some embodiments, the bispecific antibody comprises two half-antibodies, wherein each half-antibody comprises a single heavy chain variable region and optionally at least part of the heavy chain constant region, as well as a single light chain variable region and optionally at least part of the light chain constant region. In some embodiments, the bispecific antibody comprises two half-antibodies, wherein each half-antibody comprises a single heavy chain variable region and a single light chain variable region, and comprises no more than one single heavy chain variable region and no more than one single light chain variable region. In some embodiments, the bispecific antibody comprises two half-antibodies, wherein each half-antibody comprises a single heavy chain variable region and a single light chain variable region, and wherein the first half-antibody binds to the first antigen and not to the second antigen, and the second half-antibody binds to the second antigen and not to the first antigen.

The term "complementarity determining region" or "CDR region" or "CDR" (used interchangeably with hypervariable region "HVR" herein) refers to a region in an antibody variable domain that is hypervariable in sequence and forms a structure-defined loop ("a hypervariable loop") and/or contains antigen contact residues ("antigen contact points"). CDRs are mainly responsible for binding to an antigen epitope. In the present invention, the three CDRs of the heavy chain are referred to as HCDR1, HCDR2 and HCDR3, and the three CDRs of the light chain are referred to as LCDR1, LCDR2, and LCDR3.

It should be noticed that the boundaries of the CDRs of the variable regions of the same antibody obtained based on different numbering systems may be different. That is, the CDR sequences of the same antibody variable region defined under different numbering systems can be different. Therefore, for an antibody limited by specific CDR sequences defined according to the present invention, the scope of the antibody also encompasses antibodies whose variable region sequences comprises said specific CDR sequences but with different indicated CDR boundary from the specific CDR boundary defined in the present invention due to the application of different schemes (for example, different numbering system rules or combinations thereof).

Covalent linkage means that two Fc chains, or any one of the Fc chains and an antigen-binding functional region linked thereto in a heterodimeric bispecific antibody are linked with each other via a covalent bond to form a single molecule. Among them, the Fc chains comprise a first antigen-binding functional region and a second antigen-binding functional region linked via one or more covalent bonds (e.g., a disulfide bond); the first and second Fc chains are linked to an antigen-binding functional region via a covalent linkage (e.g., an imine or amide linkage), respectively.

An antigen-binding functional region is a region which can specifically interact with a target molecule, such as an antigen, and whose action is highly selective, and a sequence that recognizes one target molecule is generally unable to recognize other molecular sequences. A representative antigen-binding functional region includes an antibody variable region, a structural variant of an antibody variable region, a receptor binding domain, a ligand binding domain, or an enzyme binding domain.

One or more disulfide bond inter-chain linkages refer to the formation of a heterodimeric fragment by linkages between the first Fc chain and the second Fc chain via one or more disulfide bonds. In the present invention, one or more disulfide bonds may be formed when the first Fc chain and the second Fc chain or the first Fc chain and the second Fc chain and the antigen-binding functional regions linked thereto are synthesized in a same cell, or may be formed by *in vitro* reduction-oxidation after the first Fc chain and the second Fc chain or the first Fc chain and the second Fc chain and the antigen-binding functional regions linked thereto are synthesized in different cells, respectively.

The first Fc chain and the second Fc chain refer to forming a binding fragment through a covalent linkage, wherein the covalent linkage comprises a disulfide bond, and each chain comprises at least one portion of the heavy chain constant region of an immunoglobulin; and the first Fc chain and the second Fc chain are different from each other in their amino acid sequences, and comprise at least one different amino acid. As to the first and second Fc chains of the present invention, a strong, mutual, repulsive force exists between identical chains, and attractive force exists between different chains. Accordingly, the first and second Fc chains, or the first and second Fc chains and the antigen-binding functional regions linked thereto have a tendency to undergo heterodimeric formation, when co-expressed in a cell. When the first and second Fc chains, or the first and second Fc chains and the antigen binding domain linked thereto are expressed in two host cells, respectively, the first Fc chains, or the first Fc chain and the antigen binding domain linked thereto have no tendency to undergo homodimeric formation, and the second Fc chains, or the second Fc chain and the antigen binding domain linked thereto have no tendency to undergo homodimeric formation. In the present invention, when the first and second Fc chains, or the first and second Fc chains and the antigen-binding functional regions linked thereto are expressed in two host cells, respectively, and a reducing agent is present, a percentage of homodimers is less than 50%, that is, a percentage of monomers (one Fc chain or one Fc chain and one antigen-binding functional region linked thereto) is above 50%.

An immunoglobulin has a symmetrical structure having four polypeptide chains, wherein two chains are identical heavy chains which are relatively long and have a relatively high molecular weight, each including 450-550 amino acid residues and having a relative molecular weight of 55000-70000 Da; and the other two chains are identical light chains (L chains) which are relatively short and have a relatively low molecular weight, including about 210 amino acid residues and having a relative molecular weight of about 24000 Da. Sequences of about 110 amino acids in length near the N-termini of the heavy and light chains of an immunoglobulin are highly variable, and are called variable regions (V regions), while the rest amino acid segments near the C-termini thereof are relatively stable and called constant regions (C regions). Each heavy chain of the antibody consists of a heavy chain variable region (referred to as VH herein) and a heavy chain constant region (referred to as CH herein), and each light chain consists of a light chain variable region (referred to as VL herein) and a light chain constant region (referred to as CL herein). The variable region in the heavy chain occupies approximately 1/4 of the length of the heavy chain, and the constant region occupies approximately 3/4 of the length of the heavy chain. The known five types of Igs are IgG (γ), IgA (α), IgD (δ), IgM (µ) and IgE (ε). Among them, the first three types of Igs have three constant regions in the H chain, namely, CH1, CH2 and CH3. The latter two types of Igs (IgM and IgE) have one VH region and four constant regions in the H chain, namely CH1 to CH4. The constant regions are not only the framework of an immunoglobulin molecule but also one of the regions activating immune responses. Although embodiments of the invention relate to IgG, those skilled in the art would have recognized that the class of the antibodies of the invention can be switched by known methods, if desired. For example, an antibody of the invention that is initially IgM can be class-switched to an IgG antibody of the invention. In addition, class switching techniques can be used to convert one IgG subclass to another, such as from IgG1 to IgG2. Thus, the effector functions of the antibodies of the invention can be switched by isotypes to, for example, IgG1, IgG2, IgG3, IgG4, IgD, IgA, IgE or IgM antibodies for various therapeutic uses. In one embodiment, the antibody of the invention is an IgG1 antibody, such as IgG1, κ,.

A part of the constant region of the present disclosure includes at least an interaction region of the first Fc chain and the second Fc chain, and in the case of IgG, this region is part of amino acids located in the CH3 domain and includes at least GLN 347, TYR 349, THR 350, LEU 351, SER 354, ARG 355, ASP 356, GLU 357, LYS 360, SER 364, THR 366, LEU 368, LYS 370, ASN 390, LYS 392, THR 394, PRO 395, VAL 397, ASP 399, SER 400, PHE 405, TYR 407, LYS 409, and LYS 439.

The first Fc chain and the second Fc chain each are linked to an antigen-binding functional region via a covalent bond or a linker, meaning that the first Fc chain and the second Fc chain each are linked to an antigen-binding fragment of an antibody, or a single chain antibody capable of recognizing an antigen, or other antibody fragment variant capable of recognizing an antigen, or a receptor capable of recognizing a ligand, or a ligand capable of recognizing a receptor via a covalent bond or a linker. A covalent bond is one of chemical bonds, in which two or more atoms together use their outer electrons, to reach electronic saturation state in an ideal situation, thus forming a relatively stable chemical structure named a covalent bond. In other words, a covalent bond is an interaction between atoms by sharing their electrons. The atoms of the same element or different elements can both bind to each other via covalent bonds. The covalent bonds between the first Fc chain and the second Fc chain of the invention include, but are not limited to, an amide bond formed by carboxyl dehydration reaction between an amino group of an amino acid molecule and a carboxyl group of another amino acid molecule, or an amide bond or imine bond formed between an aldehyde group of ethylene glycol or polyethylene glycol or other compound or a polymer thereof and an amino group of an amino acid molecule; wherein the linker is one stretch of amino acid sequence or one compound or one multimer of one compound capable of linking two polypeptide chains via covalent bonds, wherein the one stretch of amino acid sequence includes, but is not limited to, a small peptide, such as GGGGSGGGGSGGGGS, which links together the first Fc chain or the second Fc chain, and a single-chain antibody capable of recognizing an antigen, or other antibody fragment structural variant capable of recognizing an antigen via amide bonds.

The first Fc chain and the second Fc chain have a tendency to undergo heterodimeric formation, rather than a tendency to undergo homodimeric formation, which means that as in the first Fc chain and the second Fc chain, a repulsive force exists between identical polypeptide chains and an attractive force exists between different polypeptide chains, and therefore, the first Fc chain and the second Fc chain, or the first and second Fc chains and the antigen-binding functional regions linked thereto have a tendency to undergo heterodimeric formation, when co-expressed in a cell. When the first Fc chain and the second Fc chain, or the first and second Fc chains and the antigen-binding functional regions linked thereto are expressed in two host cells, respectively, the first Fc chains, or the first Fc chain and the antigen-binding functional region linked thereto have no tendency to undergo homodimeric formation, and the second Fc chains, or the second Fc chain and the antigen-binding functional region linked thereto also have no tendency to undergo homodimeric formation.

The Kabat EU index numbering system refers to a method used by Kabat to assign a number for each amino acid of an antibody sequence, and this method for assigning a number for each residue has become a standard method in the art. The Kabat protocol can be applied to other antibodies that are not in its research. Based on conserved amino acids, the target antibody is aligned with one of the consensus sequences identified by Kabat.

The Fc domain refers to a fragment crystallizable (Fc), corresponds to CH2 and CH3 domains of an Ig, and is a site where an Ig interacts with an effector molecule or a cell.

IgG is an abbreviation of immunoglobulin G (IgG), and is the main type of antibodies in serum. Human IgG has four subclasses, namely IgG1, IgG2, IgG3 and IgG4, based on antigenic differences in r-chains in the IgG molecules.

A half-antibody molecule refers to a structure formed by one heavy chain and one light chain of an antibody, wherein the heavy chain and the light chain may be linked via or not via a covalent bond, and is a monovalent antibody structure recognizing an antigen.

A Fab fragment is a molecule-recognizing sequence, i.e., a fragment of antigen binding (Fab), and corresponds to two arms of an antibody molecule, consisting of a complete light chain and VH and CH1 domains of a heavy chain. scFv, a molecule- recognizing sequence, is a structural variant of an antibody fragment obtained by genetic engineering modification of a light chain variable region and a heavy chain variable region of an antibody. An extracellular region of a membrane receptor is a molecule-recognizing sequence, and the membrane receptor generally comprises an extracellular region located outside the cell that recognizes and binds to the corresponding antigen or ligand, a transmembrane region that anchors the receptor onto the cell surface, and an intracellular region in a cell that has kinase activity or transfers a signal. A ligand of the cell membrane receptor refers to a protein, a small peptide or a compound that may be recognized and bound by the extracellular region of a membrane receptor. Cytokines are low-molecular weight soluble proteins that are produced due to the induction of various types of cells by immunogens, mitogens, or other stimulants, and have various functions such as regulations of innate immunity and adaptive immunity, hematopoiesis, cell growth, APSC pluripotent cell, and damage tissue repair, etc. Cytokines may be classified into interleukins, interferons, tumor necrosis factor superfamilies, colony stimulating factors, chemokines, growth factors, etc. A protein expression tag means an amino acid sequence added to the N-terminus or C-terminus of a target protein, and may be a small peptide or a long amino acid sequence. Addition of a tag may be advantageous for correct folding of proteins, isolation, and purification of proteins, and decreasing intracellular degradation of proteins. Tags frequently used may include, but not limited to HA, SUMO, His, GST, GFP and Flag.

The bispecific antibody in a heterodimeric form of the present invention may have one or more substitutions, deletions, additions and/or insertions. For example, certain amino acids can substitute other amino acids in the protein structure without significant loss of the ability to bind to other polypeptides (such as antigens) or cells. Since the binding ability and protein properties determine the bioactivities and functions of a protein, substitutions of certain amino acid sequences can be made in the protein sequence without significant loss of their biological efficacy or activities.

In many cases, a polypeptide variant contains one or more conservative substitutions. The "conservative substitution" means that in which an amino acid in the polypeptide is replaced by another amino acid with similar properties, such that the skilled in the peptide chemistry field would anticipate that the secondary structure and hydrophilic property of the polypeptide are substantially unchanged.

Amino acid substitutions are generally based on the relative similarity of the amino acid side chain substituents, such as their hydrophobicity, hydrophilicity, charges, size, etc. Exemplary substitutions that take various aforementioned characteristics into account are well known to those skilled in the art and include: arginine and lysine; glutamic acid and aspartic acid; serine and threonine; glutamine and asparagine; and valine, leucine and isoleucine.

As used herein, the term "identity" has the meaning generally known in the art. Those skilled in the art are also familiar with the rules and criteria for determining the identity between different sequences, and "identity" refers to the percentage of residues in a variant polynucleotide or polypeptide sequence that are identical to that of a non-variant sequence after aligning the sequences and introducing gaps (if necessary, in order to obtain the maximum percent homology). In the present invention, under the condition that the identity restriction is met, the obtained variant sequence also needs to have the biological activities possessed by the parent sequence. The skilled in the art would have well known the methods and means for screening variant sequences according to the above-described activities. Such variant sequences could be readily obtained by those skilled in the art in the light of the disclosure of the present application. In a specific embodiment, the polynucleotide and polypeptide variants have at least about 70%, at least about 75%, at least about 80%, at least about 90%, at least about 95%, at least about 98%, or at least about 99%, or at least about 99.1%, 99.2%, 99.3%, 99.4%, 99.5%, 99.6%, 99.7%, 99.8%, or 99.9% polynucleotide or polypeptide identity with the polynucleotide and polypeptide described herein. Due to the redundancy of the genetic codons, there will be variants of these sequences encoding the same amino acid sequence.

In another embodiment of the present invention, a polynucleotide composition capable of hybridizing with the polynucleotide sequence or the fragment or the complementary sequence thereof provided in the present invention under moderately to highly stringent conditions is provided. The hybridization techniques are well known in the art of molecular biology. For the purposes of illustration, suitable moderately stringent condition for testing the hybridization of the polynucleotide of the present invention with other polynucleotides include pre-washing with a solution of 5×SSC, 0.5% SDS, and 1.0 mM EDTA (pH 8.0); hybridizing at 50-60°C, 5×SSC, overnight; and then washing twice with 2×, 0.5× and 0.2× SSC containing 0.1% SDS at 65°C for 20 minutes. Those skilled in the art would recognize that the stringency of hybridization may be easily manipulated, for example, by changing the salt content of the hybridization solution and/or the temperature during hybridization. For example, in another embodiment, suitable highly stringent hybridization conditions include the conditions described above, except that the hybridization temperature is increased, for example, to 60-65°C or 65-70°C.

The host cell of the present invention may be any cell used for exogenous gene expression, including but not limited to *Escherichia coli,* yeast, insect cells, plant cells, and mammalian cells.

The vector of the present invention includes a vector that may replicate in any type of cells or organisms, including, for example, plasmids, bacteriophages, cosmids, and mini-chromosomes. In some embodiments, the vector comprising the polynucleotide of the present invention is a vector suitable for propagation or replication of the polynucleotide, or a vector suitable for expressing the polypeptide of the present invention. Such vectors are known in the art and are commercially available.

The "vector" includes shuttle vectors and expression vectors. In general, a plasmid construct also includes an origin of replication (such as the ColE1 origin of replication) and a selectable marker (such as ampicillin or tetracycline resistance) for plasmid replication and selection in bacteria, respectively. The "expression vector" refers to a vector containing the control sequences or regulatory elements required for expressing the antibody of the present invention, including antibody fragments, in bacteria or eukaryotic cells.

The vector of the present invention may be any vector used for the exogenous gene expression, including but not limited to plasmid vectors, which contains at least an origin of replication, a promoter, a target gene, a multiple cloning site, and a gene of the selectable marker, and preferably, the vector of the present invention includes but is not limited to modified plasmid vector obtained based on pCDNA, such as XOGC vector.

The subjects of the present invention include birds, reptiles, mammals, etc. Preferably, the mammals include rodents and primates, and preferably, the primates include humans.

The scope of the diseases involved in the present invention includes but is not limited to tumors. Preferably, the tumors include: leukemia, lymphoma, myeloma, brain tumor, head and neck squamous cell cancer, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell cancer, melanoma, small cell lung cancer and bone cancer.

A pharmaceutically acceptable carrier refers to a pharmaceutical carrier commonly used in the pharmaceutical art, such as diluents, excipients and water, etc.; fillers such as starch, sucrose, lactose, microcrystalline cellulose, etc.; binders such as cellulose derivatives, alginates, gelatin and polyvinylpyrrolidone; wetting agents such as glycerin; disintegrating agents such as sodium carboxymethyl starch, hydroxypropyl cellulose, croscarmellose, agar, calcium carbonate and sodium hydrogencarbonate; absorption enhancers such as quaternary ammonium compounds; surfactants such as cetanol, sodium lauryl sulfate; adsorption carriers such as kaolinite and bentonite; lubricants such as talc, calcium and magnesium stearate, micronized silica gel and polyethylene glycol, etc.. In addition, other adjuvants such as flavoring agents and sweetening agents may also be added into the composition.

The present invention will be further illustrated with reference to the following nonlimiting examples. Those skilled in the art would know that many modifications may be made to the present invention without departing from the spirit of the present invention, and such modifications also fall within the scope of the present invention.

The following experimental methods are all conventional methods unless otherwise specified, and all the experimental materials used are easily available from commercial companies unless otherwise specified. The various antibodies used in the following examples of the present invention are all standard antibodies commercially available.

### Examples

### Example 1. Construction of the vector for anti-PD-L1/anti-CD47 heterodimeric antibody molecule

The XOGC expression vectors (see PCT publication no. WO2008/048037) containing the heavy chain or light chain of the anti-human PD-L1 antibody were constructed, respectively. The nucleotide sequence of the light chain variable region is set forth in SEQ ID NO. 1, and the amino acid sequence thereof is set forth in SEQ ID NO: 2; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 5, and the amino acid sequence thereof is set forth in SEQ ID NO: 6; the nucleotide sequence of the heavy chain constant region is set forth in SEQ ID NO. 7 or 9, and the amino acid sequence thereof is set forth in SEQ ID NO: 8 or 10. Fragments comprising restriction sites, a signal peptide sequence and the light chain variable region-encoding sequence of the anti-PD-L1 antibody were synthesized by Genewiz Ltd. The synthesized vector comprising the fragment of interest and XOGC vector comprising a light chain constant region-encoding sequence were double digested with BamHI (Thermo, catalog number FD0054) and Pfl23II (Thermo, catalog number FD0854), and the digestion reaction system was: 10× buffer 6 µl, 2 µl BamHI and 2 µl Pfl23II, the vector 40µl, H₂O 10µl, and the digestion system reaction was conducted at 37°C for 1 hour. The digested products were ligated using T4DNA ligase (Thermo, catalog number EL0011), and the reaction system was: 10×ligase buffer 1µl, the ligase 0.5µl, the light chain variable region fragments obtained from gel recovery 7.5µl, the XOGC vector comprising a ligated light chain constant region-encoding sequence obtained from gel recovery 1µl, and the ligation reaction was conducted at 22°C for 30min. The ligation products were transformed into *E*. *coli* competent cell DH5α (Tiangen, CB104, the same hereinafter). Fragments comprising restriction sites, a signal peptide sequence and the heavy chain variable region-encoding sequence of the anti-PD-L1 antibody were synthesized by Genewiz Ltd. The synthesized vector comprising the fragment of interest and XOGC vector comprising a heavy chain constant region-encoding sequence were double digested with HindIII (Thermo, catalog number FD0505) and NheI (Thermo, catalog number FD0973), and the digestion reaction system was: 10× buffer 6 µl, 2 µl HindIII and 2 µl NheI, the vector 40µl, H₂O 10µl, and the digestion system reaction was conducted at 37°C for 1 hour. The digested products were ligated using T4DNA ligase (Thermo, catalog number EL0011), and the reaction system was: 10×ligase buffer 1µl, the ligase 0.5µl, the heavy chain variable region fragments obtained from gel recovery 7.5µl, the XOGC vector comprising a ligated heavy chain constant region-encoding sequence obtained from gel recovery 1µl, and the ligation reaction was conducted at 22°C for 30min. The ligation products were transformed into *E. coli* competent cell DH5α (Tiangen, CB104, the same hereinafter). The XOGC expression vectors for the anti-human PD-L1 antibody heavy chain and light chain were obtained and used to express the anti-human PD-L1 antibody heavy chain and light chain in eukaryotic cells, respectively.

The XOGC expression vectors containing the sequences encoding the heavy chain and light chain variable regions of the anti-human CD47 murine antibody were also constructed in the present invention respectively. The nucleotide sequence of the light chain variable region is set forth in SEQ ID NO. 11, and the amino acid sequence thereof is set forth in SEQ ID NO: 12; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 13, and the amino acid sequence thereof is set forth in SEQ ID NO: 14; the nucleotide sequence of the heavy chain constant region is set forth in SEQ ID NO. 15, and the amino acid sequence thereof is set forth in SEQ ID NO: 16. The XOGC expression vectors for the anti-human CD47 antibody heavy chain and light chain were obtained and used to express the heavy chain and light chain of the anti-human CD47 antibody CD47-18-5 in eukaryotic cells, respectively.

The XOGC expression vectors containing the sequences encoding the heavy chain and light chain variable regions of the anti-human CD47 murine antibody CD47-18-10 were also constructed in the present invention respectively. The nucleotide sequence of the light chain variable region is set forth in SEQ ID NO. 17, and the amino acid sequence thereof is set forth in SEQ ID NO: 18; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 19, and the amino acid sequence thereof is set forth in SEQ ID NO: 20; the nucleotide sequence of the heavy chain constant region is set forth in SEQ ID NO. 15, and the amino acid sequence thereof is set forth in SEQ ID NO: 16. The XOGC expression vectors for the anti-human CD47 antibody heavy chain and light chain were obtained and used to express the heavy chain and light chain of the anti-human CD47 antibody CD47-18-10 in eukaryotic cells, respectively.

The XOGC expression vectors containing the sequences encoding the heavy chain and light chain variable regions of the humanized sequences z3 of the anti-human CD47 antibody CD47-18-5 were also constructed in the present invention respectively. The nucleotide sequence of the light chain variable region is set forth in SEQ ID NO. 21, and the amino acid sequence thereof is set forth in SEQ ID NO: 22; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 23, and the amino acid sequence thereof is set forth in SEQ ID NO: 24; the nucleotide sequence of the heavy chain constant region is set forth in SEQ ID NO. 15, and the amino acid sequence thereof is set forth in SEQ ID NO: 16. The XOGC expression vectors for the anti-human CD47 antibody heavy chain and light chain were obtained and used to express the heavy chain and light chain of the humanized sequences z3 of the anti-human CD47 antibody CD47-18-5 in eukaryotic cells, respectively.

The XOGC expression vectors containing the sequences encoding the heavy chain and light chain variable regions of the humanized sequences z4 of the anti-human CD47 antibody CD47-18-5 were also constructed in the present invention respectively. The nucleotide sequence of the light chain variable region is set forth in SEQ ID NO. 25, and the amino acid sequence thereof is set forth in SEQ ID NO: 26; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 27, and the amino acid sequence thereof is set forth in SEQ ID NO: 28; the nucleotide sequence of the heavy chain constant region is set forth in SEQ ID NO. 15, and the amino acid sequence thereof is set forth in SEQ ID NO: 16. The XOGC expression vectors for the anti-human CD47 antibody heavy chain and light chain were obtained and used to express the heavy chain and light chain of the humanized sequences z4 of the anti-human CD47 antibody CD47-18-5 in eukaryotic cells, respectively.

The XOGC expression vectors containing the sequences encoding the heavy chain and light chain variable regions of the humanized sequences z7 of the anti-human CD47 antibody CD47-18-5 were also constructed in the present invention respectively. The nucleotide sequence of the light chain variable region is set forth in SEQ ID NO. 29, and the amino acid sequence thereof is set forth in SEQ ID NO: 30; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 31, and the amino acid sequence thereof is set forth in SEQ ID NO: 32; the nucleotide sequence of the heavy chain constant region is set forth in SEQ ID NO. 15, and the amino acid sequence thereof is set forth in SEQ ID NO: 16. The XOGC expression vectors for the anti-human CD47 antibody heavy chain and light chain were obtained and used to express the heavy chain and light chain of the humanized sequences z7 of the anti-human CD47 antibody CD47-18-5 in eukaryotic cells, respectively.

The XOGC expression vectors containing the sequences encoding the heavy chain and light chain variable regions of the humanized sequences z11 of the anti-human CD47 antibody CD47-18-5 were also constructed in the present invention respectively. The nucleotide sequence of the light chain variable region is set forth in SEQ ID NO. 33, and the amino acid sequence thereof is set forth in SEQ ID NO: 34; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 35, and the amino acid sequence thereof is set forth in SEQ ID NO: 36; the nucleotide sequence of the heavy chain constant region is set forth in SEQ ID NO. 15, and the amino acid sequence thereof is set forth in SEQ ID NO: 16. The XOGC expression vectors for the anti-human CD47 antibody heavy chain and light chain were obtained and used to express the heavy chain and light chain of the humanized sequences z11 of the anti-human CD47 antibody CD47-18-5 in eukaryotic cells, respectively.

### Example 2. Expression of the anti-PD-L1/anti-CD47 heterodimeric antibody molecule

The expression vectors containing the heavy or light chains of the anti-human PD-L1 antibody were co-transfected into ExpiCHO cells (ExpiCHO^{™} Cells, catalog number A29127, invitrogen). In addition, various expression vectors containing the heavy or light chains of the anti-human CD47 antibodies were also co-transfected into ExpiCHO cells.

The cells were seeded at a seeding density of 3.5×10⁶ cells/mL the day before transfection. On the day of transfection, the cells were diluted with fresh ExpiCHO expression medium (ExpiCHO^{™} Expression Medium, catalog number A29100-01, invitrogen) at a dilution density of 6×10⁶ cells/mL. The plasmids were taken out according to the transfection volume to achieve a final concentration of 0.5 µg/ml. The plasmids were diluted to 4% of the transfection volume using OptiPRO^{™} SFM medium (OptiPRO^{™} SFM, catalog number 12309-019, invitrogen), and mixed homogeneously by inversion. The ExpiFectamine^{™} transfection reagent (ExpiFectamine^{™} CHO Transfection Kit, catalog number A29129, invitrogen) in an amount of 6.4 times of the plasmid amount was taken out and was diluted to 4% of the transfection volume using OptiPRO^{™} SFM medium, and mixed homogeneously by inversion. The diluted transfection reagent was added to the diluted plasmids, mixed gently, leaved at room temperature for 1 to 5 min, and slowly added dropwise into the cells. The cells were then placed into a cell incubator (8% CO₂ concentration), and incubated at 37°C for 20 hours on a shaker at 120rpm. The ExpiCHO^{™} Enhancer (ExpiFectamine^{™} CHO Transfection Kit, catalog number A29129, invitrogen) in an amount of 0.006 times of the transfection volume and the ExpiCHO^{™} Feed (ExpiCHO^{™} Feed, catalog number A29101-02, invitrogen) in an amount of 0.24 times of the transfection volume were slowly added into the cells. The cells were placed in a shaker at 120 rpm and incubated at 32°C. The supernatant of the cell culture was collected by centrifugation 10 days after transfection.

The expression levels were determined by an ELISA assay. A filtration step with 0.2µm filter membrane was performed to remove the precipitates before applying the chromatography column purification. This step was performed at 4°C.

### Example 3. Purification of the expression products of the anti-PD-L1/anti-CD47 heterodimeric antibody molecule

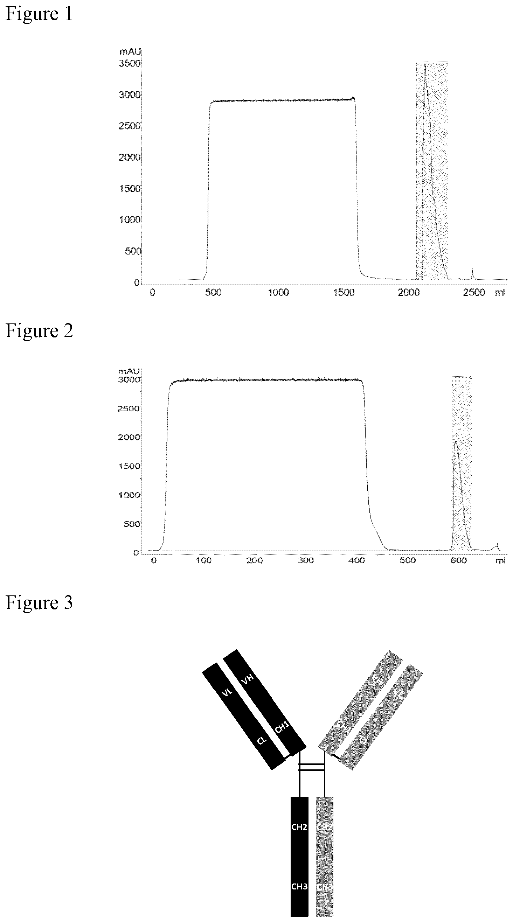

The purification was performed at 4 °C using AKTA explorer 100 protein purification system (GE Healthcare) and affinity chromatography column MabSelect SuRe (16 mm I.D., 22 ml, GE Healthcare). The chromatography column was first equilibrated with mobile phase A (20 mM sodium phosphate buffer, pH 7.4), the cell supernatant as treated above was loaded once the baseline becomes stable, and then equilibrated with mobile phase A. The samples were the anti-PD-L1 expression products and the anti-CD47 expression products, respectively. Thereafter, the column was firstly washed with 5 column volumes of mobile phase B (mobile phase A containing 1 M sodium chloride), and then washed with 2 column volumes of mobile phase A, and then eluted with 5 column volumes of mobile phase C (100 mM glycine, PH 3.3), and the elution peak collected was the target protein peak. All the flow rates of the above elution steps are 5 ml/min. The chromatogram of the elution peak of the anti-PD-L1 expression products is shown in Figure 1, and the elution peak of the anti-CD47 expression products (consisting of the heavy and light chains of the humanized sequences z11 of the antibody CD47-18-5) is shown in Figure 2. The elution peak marked (the gray area in the figure) was collected, adjusted to pH 7.2 by dropwise addition of 1 M Tris solution, and sterile filtered and stored at 2~8°C.

### Example 4. Preparation and purification of the anti-PD-L1/anti-CD47 heterodimeric antibody molecule

The structure of the anti-PD-L1/anti-CD47 heterodimeric antibody molecule is shown in Figure 3.

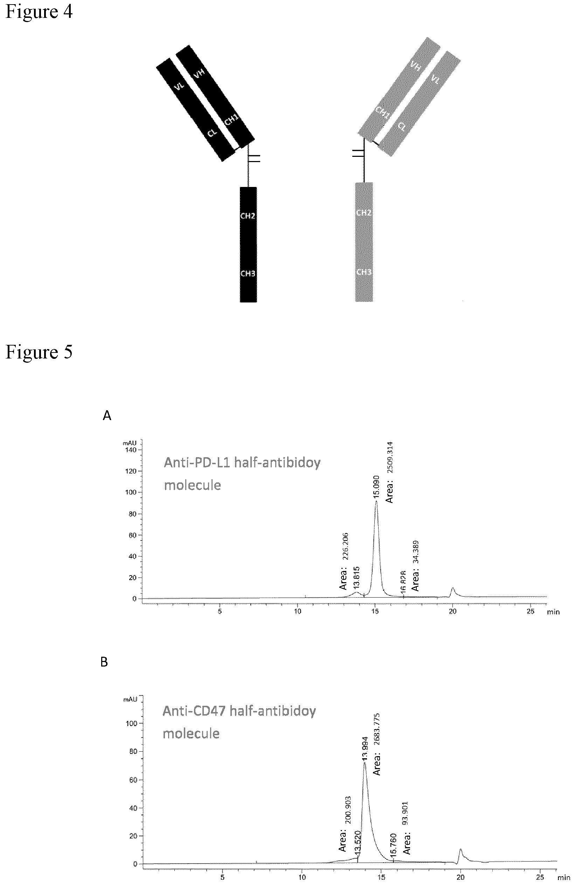

The products purified by the MabSelect SuRe (16 mm I.D., 22 ml, GE Healthcare) method described above were subjected to *in vitro* reconstitution to obtain heterodimers. First, the collected protein solutions after the purification described above were subjected to a DTT reduction process to open up the disulfide bonds, and the disulfide bonds in the hinge region of the antibody homodimeric molecules contained in the anti-PD-L1 and anti-CD47 products were also opened, to form half-antibody molecules containing one heavy chain and one light chain, of which the structure is shown in Figure 4. The reduced sample was analyzed by SEC-HPLC (TOSOH, TSKgel superSW3000) with mobile phase buffer containing 1mM DTT reducing agent. The results are shown in Figure 5 (panels A and B), respectively. The ratios of the anti-PD-L1 and the anti-CD47 homodimeric molecules are both less than 20%, and the ratio of the half-antibody molecules is greater than 80%.

Next, the reduced anti-PD-L1 and anti-CD47 half-antibody molecules were mixed in an equimolar ratio, and the reconstitution reaction was carried out at 4 ° C for 24 hours. During the reconstitution process, the anti-PD-L1 and the anti-CD47 half-antibody molecules formed heterodimeric bispecific antibody containing both the anti-PD-L1 and the anti-CD47 half-antibody molecules through non-covalent force between CH2 and CH3 domains. The protein solution was then subjected to ultrafiltration concentration through an ultrafiltration concentration tube (nominal molecular weight cut-off of 10KDa), and the solution was replaced with phosphate buffer (PBS, pH = 7.4) to stop the reduction, and then was subjected to oxidation reaction with air or an oxidizing agent, to re-form the disulfide bonds in the heterodimeric bispecific antibody.

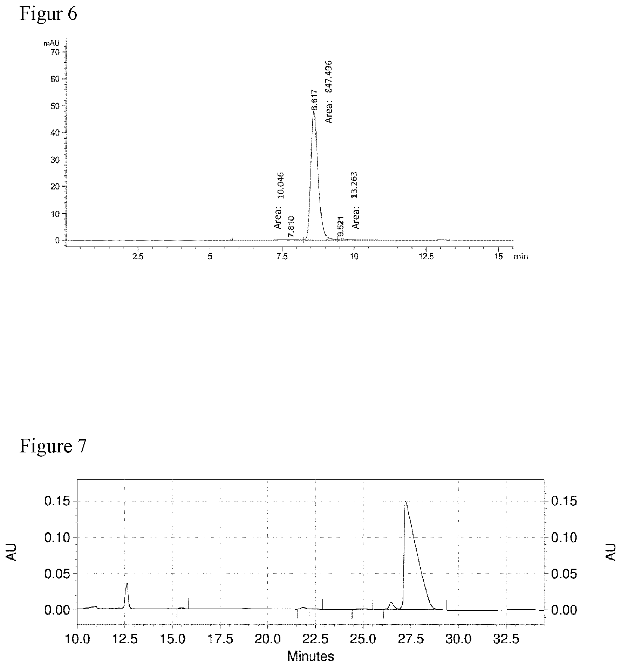

The heterodimeric antibody molecules obtained above by reduction and oxidation of the anti-PD-L1 and anti-CD47 expression products were subjected to ultrafiltration concentration through an ultrafiltration concentration tube (nominal molecular weight cut-off of 10KDa), and the solution was replaced with 10 mM sodium phosphate buffer, pH 6.0. The purification was performed at room temperature using AKTA explorer 100 protein purification system (GE Healthcare) and ion chromatography column Source 15S (16 mm I.D., 22ml, ThermoFisher). First, the chromatography column was equilibrated with mobile phase A (10 mM sodium phosphate, pH 7.0). The protein solution as treated above was loaded once the baseline becomes stable with a flow rate of 5 ml/min, and then equilibrated with mobile phase A. Next, the column was washed with 20 column volumes of a gradient from A (10 mM sodium phosphate, pH 5.8) to B (10 mM sodium phosphate, pH 5.8) (0% B-100% B, 170 min, flow rate 2 ml/min). The main elution peak was collected, and the collected protein solution was subjected to ultrafiltration concentration through an ultrafiltration concentration tube (nominal molecular weight cut-off of 10KDa), and then the solution was replaced with a phosphate solution (PBS, pH 7.4); and sterilized through filtration, and then stored at 4°C. The purity of the purified anti-PD-L1/anti-CD47 heterodimeric antibody molecules BH3012h (wherein the anti-CD47 antibody portion thereof comprises the heavy and light chain variable region sequences of the humanized sequences z11 of the antibody CD47-18-5) analyzed by SEC-HPLC was 97.3%, as shown in Figure 6; CE analysis was also performed, and the purity is 95.3%, as shown in Figure 7. The results of other anti-PD-L1/anti-CD47 heterodimeric antibody molecules such as BH3014 (wherein the anti-CD47 antibody portion thereof comprises the heavy and light chain variable region sequences of the humanized sequences z3 of the antibody CD47-18-5), BH3015 (wherein the anti-CD47 antibody portion thereof comprises the heavy and light chain variable region sequences of the humanized sequences z4 of the antibody CD47-18-5), BH3016 (wherein the anti-CD47 antibody portion thereof comprises the heavy and light chain variable region sequences of the humanized sequences z7 of the antibody CD47-18-5), the anti-PD-L1/anti-CD47 heterodimeric bispecific antibody 18-5 (wherein the anti-CD47 antibody portion thereof comprises the heavy and light chain variable region sequences of the murine antibody CD47-18-5), and the anti-PD-L1/anti-CD47 heterodimeric bispecific antibody 18-10 (wherein the anti-CD47 antibody portion thereof comprises the heavy and light chain variable region sequences of the murine antibody CD47-18-10) are similar to those of BH3012h.

### Example 5. Target binding activity of the anti-PD-L1/anti-CD47 heterodimeric antibody

Enzyme-linked immunosorbent assay (ELISA) was used to determine the binding ability of the anti-PD-L1/anti-CD47 heterodimeric antibody to a single antigen. The detailed process is as follows: a 96-well high-adsorption ELISA plate (Costar, catalog number 42592) was coated with 1 µg/mL recombinant human PD-L1 (Sino Biological Inc., catalog number 10084-H08H) or human CD47 (ACROBiosystems, catalog number CD7-H5227) at 100 µL per well using carbonate buffer solution, pH=9.6 at 4°C overnight. The plate was washed 5 times with PBST, and then blocked using PBST with 1% BSA, 300 µL/well, and incubated at 25°C for 1 hour, and then washed 5 times with PBST. The heterodimeric antibody samples serially diluted in PBST with 1% BSA and the control were added, 100 µL per well and incubated at 25°C for 1 hour. The plate was washed 5 times with PBST. The horseradish peroxidase-labeled anti-human IgG antibody (Chemicon, catalog number AP309P) diluted 1:10000 in PBST with 1% BSA was then added, 100 µL per well, and incubated at 25°C for 1 hour. The plate was washed 5 times with PBST. The colorimetric substrate TMB was added, 100µL/well, and developed at room temperature for 10 minutes. 1M H₂SO₄ was added, 100 µL/well, to stop the development. The absorbance at 450 nm was read on a microplate reader.

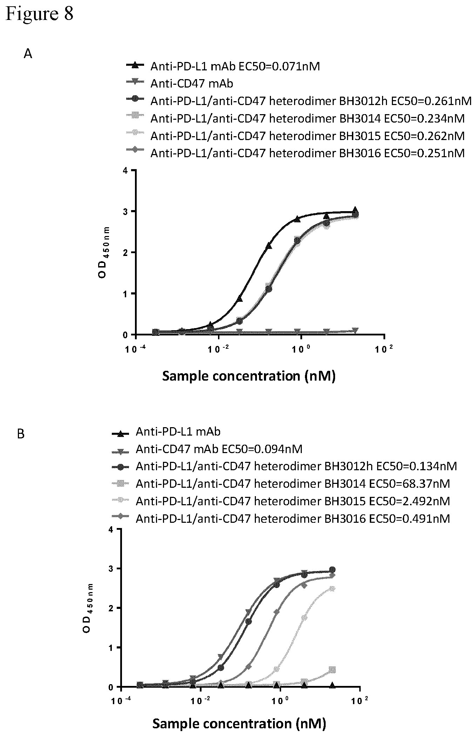

As shown by the results of Figure 8 (panel A), all the anti-PD-L1/anti-CD47 heterodimeric antibodies BH3012h, BH3014, BH3015 and BH3016 have a high affinity with PD-L1 (their activities are comparable), which is slightly lower than the antigen binding activity of the PD-L1 bivalent monoclonal antibody. As shown in Figure 8 (panel B), the anti-PD-L1/anti-CD47 heterodimeric antibodies BH3012h, BH3014, BH3015 and BH3016 have different affinities with CD47, wherein BH3012h has the strongest affinity, followed by BH3016, followed by BH3015, and BH3014 has the weakest binding activity.

### Example 6. Ligand-receptor binding blocking activity of the anti-PD-L1/anti-CD47 heterodimeric antibody

The detection process of the blocking activity for PD-L1 binding to PD-1 and CD80 is as follows: a 96-well high-binding ELISA plate was coated with 1 µg/mL human PD-L1-Fc (ACROBiosystems, catalog number PD1-H5258), 100 µL per well, using carbonate buffer solution, pH=9.6 at 4°C overnight, and washed 5 times with PBST. The plate was then blocked with PBST containing 1% BSA, 300 µL/well, incubated at 25°C for 1 hour, and washed 5 times with PBST. The heterodimeric antibody samples serially diluted in PBST with 1% BSA and the control were added, 50µL per well, as well as 50µL of the biotin-labeled PD-1-Fc (Beijing Hanmi Pharmaceutical Co., LTD.) at a concentration of 40nM (final concentration 20nM) or 50µL of the biotin-labeled CD80-Fc (ACROBiosystems, catalog number B71-H82F2) at a concentration of 100nM (final concentration 50nM), and the plate was incubated at 25°C for 90 minutes and washed 5 times with PBST. Streptavidin-HRP (BD Pharmingen, catalog number 554066) diluted 1:1000 in PBST with 1% BSA was then added, 100µL per well, and the plate was incubated at 25°C for 1 hour and washed 5 times with PBST. The colorimetric substrate TMB was added, 100µL/well, and developed at room temperature for 10 minutes. 1M H₂SO₄ was then added, 100µL/well, to stop the development. The absorbance at 450nm was read on a microplate reader.

The detection process of the blocking activity for CD47 binding to SIRPα is as follows: a 96-well high-binding ELISA plate was coated with 1 µg/mL recombinant human CD47-Fc (ACROBiosystems, catalog number CD7-H52A5), 100 µL per well, using carbonate buffer solution, pH=9.6 at 4°C overnight, and washed 5 times with PBST. The plate was then blocked with PBST containing 1% BSA, 300 µL/well, incubated at 25°C for 1 hour, and washed 5 times with PBST. The heterodimeric antibody samples serially diluted in PBST with 1% BSA and the control were added, 50µL per well, as well as 50µL of the biotin-labeled SIRPα (ACROBiosystems, catalog number CDA-H82F2) at a concentration of 4 nM (final concentration 2 nM), and the plate was incubated at 25°C for 90 minutes and washed 5 times with PBST. Streptavidin-HRP (BD Pharmingen, catalog number 554066) diluted 1:1000 in PBST with 1% BSA was then added, 100µL per well, and the plate was incubated at 25°C for 1 hour and washed 5 times with PBST. The colorimetric substrate TMB was added, 100µL/well, and developed at room temperature for 10 minutes. 1M H₂SO₄ was then added, 100µL/well, to stop the development. The absorbance at 450nm was read on a microplate reader.

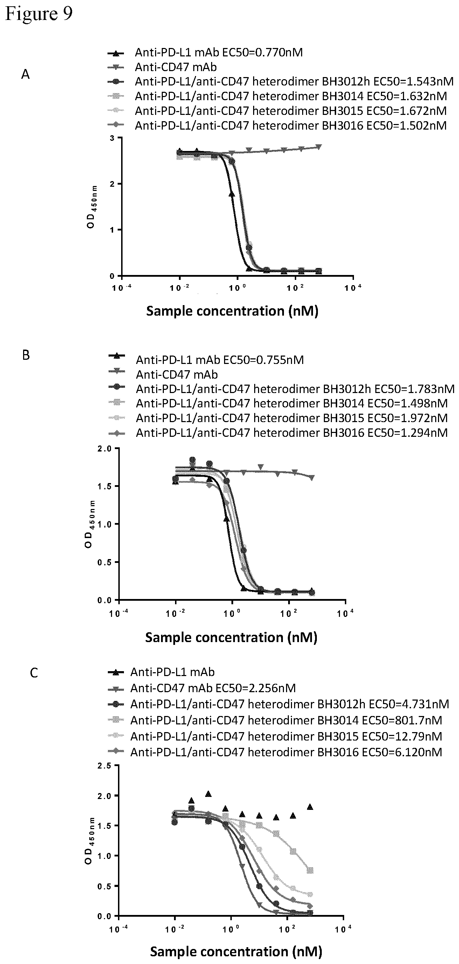

As shown by the result of Figure 9 (panel A), all the anti-PD-L1/anti-CD47 heterodimeric antibodies BH3012h, BH3014, BH3015 and BH3016 are able to block the binding of PD-L1 to PD-1 (their activities are comparable), with the activity slightly weaker than that of the anti-PD-L1 bivalent monoclonal antibody. As shown in Figure 9 (panel B), all the anti-PD-L1/anti CD47 heterodimeric antibodies BH3012h, BH3014, BH3015 and BH3016 are able to block the binding of PD-L1 to CD80, with comparable activities. As shown in Figure 9 (panel C), the anti-PD-L1/antiCD47 heterodimeric antibodies BH3012h, BH3014, BH3015 and BH3016 have different activities of blocking the binding of CD47 to SIRPα, wherein BH3012h has the strongest activity, followed by BH3016, followed by BH3015, and BH3014 has the weakest activity.

### Example 7. Binding of the anti-PD-L1/anti-CD47 heterodimeric antibody to tumor cells/red blood cells

HCC827 tumor cells express PD-L1 and CD47, but red blood cells (RBCs) express only CD47. HCC827 cells and RBCs were mixed together, and flow cytometry (FCM) was used to detect whether the heterodimer was selective for the binding to the two cells in the mixed cells.

The specific implementation process of this method is as follows. HCC827 cells (purchased from ATCC) and RBCs (collected from healthy people) were collected, washed once with cold PBS (GIBCO, Catalog number 14190-235) containing 2% FBS (Hyclone, Catalog number SH30084.03). HCC827 cells (1×10⁶ cells per tube) and RBCs (10×10⁶ cellsper tube) were mixed together, resuspended in 200 µL of cold DPBS (GIBCO, Catalog number 14190-136) containing 2% FBS, and the serially diluted heterodimeric antibody samples and controls were added therein. Flow tubes were incubated on ice for 30 minutes. The cells were washed twice with PBS containing 2% FBS. Then the pellets were resuspended in 200 µL of cold PBS containing 2% FBS and FITC-labeled anti-human IgG antibody diluted 1:1000 (Beijing Zhongshan Golden Bridge, Catalog number ZF0306), incubated on ice for 30 minutes in the dark. The cells were washed twice with PBS containing 2% FBS, then resuspended in 500 µL of cold PBS. The cell suspension obtained was detected and analyzed on a flow cytometer (BD, FACS Calibur), and the respective fluorescence intensities of the two cells in the mixed cells were read.

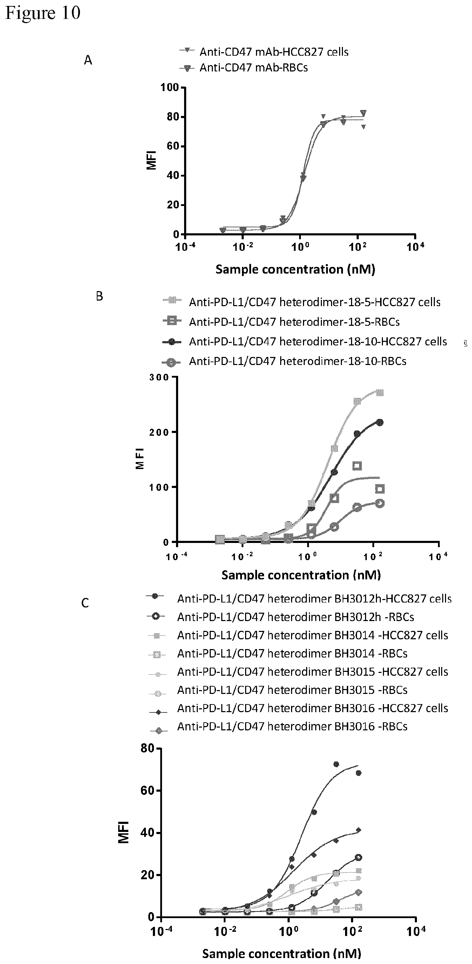

The results were shown in Figure 10. The bindings of CD47 monoclonal antibody (comprising the heavy and light chain variable region sequences of the humanized sequences z11 of the antibody CD47-18-5 and the constant region sequence of human IgG4) to HCC827 cells and RBCs mixed together are not substantially different from each other (panel A); while the anti-PD-L1/anti-CD47 heterodimeric antibody tends to bind to HCC827 cells co-expressing PD-L1 and CD47, but binds weakly or even not bind to RBCs expressing only CD47, showing the selectivity of binding (panels B and C).

### Example 8. T cell modulation activity of the anti-PD-L1/anti-CD47 heterodimeric antibody

A mixed lymphocyte reaction (MLR) was used to determine the modulation activity of the anti-PD-L1/anti-CD47 heterodimeric antibody on T cell immune response.

Obtaining of the human dendritic cells (DCs): The human PBMC cells (Lonza, Catalog number CC-2702) were thawed and collected, re-suspended in serum-free RPMI 1640 medium (GIBCO, catalog number 22400-089) at a cell density of 5×10⁶/mL and seeded in a cell culture flask, and cultured in a 37 °C CO₂ incubator for 90 minutes. Discarding the culture supernatant and suspension cells, and the adherent cells were incubated for 3 days in a complete medium (RPMI 1640 with 10% FBS) with the addition of 100ng/ml GM-CSF (Sino Biological Inc., catalog number 10015-HNAH) and 100ng/ml IL-4 (Sino Biological Inc., catalog number 11846-HNAE), and the medium was replaced, and incubated for another 3 days. Then the medium was replaced with a complete medium (RPMI 1640 with 10% FBS) containing 100ng/ml GM-CSF, 100ng/ml IL-4 and 20ng/ml TNF-α, and incubated for one day to obtain the DCs.

Obtaining of the human T cells: the human PBMC cells were thawed and collected to ensure that these PBMCs and the PBMCs for inducing the DC cells are from different individuals. The human T cells were isolated according to the instructions of the Pan T cell isolation kit (Miltenyi Biotech, catalog number 5150414820). Briefly, PBMCs were washed once with PBS, and then re-suspended at 10⁷ cells per 40µL separation buffer (PBS with 2mM EDTA, 0.5% BSA, pH=7.2) (the following amounts used are all for 10⁷ cells). 10µL Pan T cell Biotin Antibody Cocktail was added and incubated at 4°C for 5 minutes. 30µL separation buffer and 20µL Pan T cell MicroBead Cocktail were then added and incubated at 4°C for 10 minutes. The T cells were obtained after passing the MACS separation column.

The collected human DCs and human T cells were re-suspended in a complete medium (RPMI 1640 with 10% FBS) and seeded in a 96-well plate at 1×10⁴ cells/well and at 1×10⁵ cells/well, respectively, mixed and incubated. The heterodimeric antibody samples serially diluted with a complete medium and the control were added. The culture plate was placed in a CO₂ incubator at 37°C and incubated for 5 days. After the incubation, the supernatants were removed from the wells and the cytokine IFN-γ was detected according to the manual of the kit (RayBiotech, catalog number ELH- IFNg).

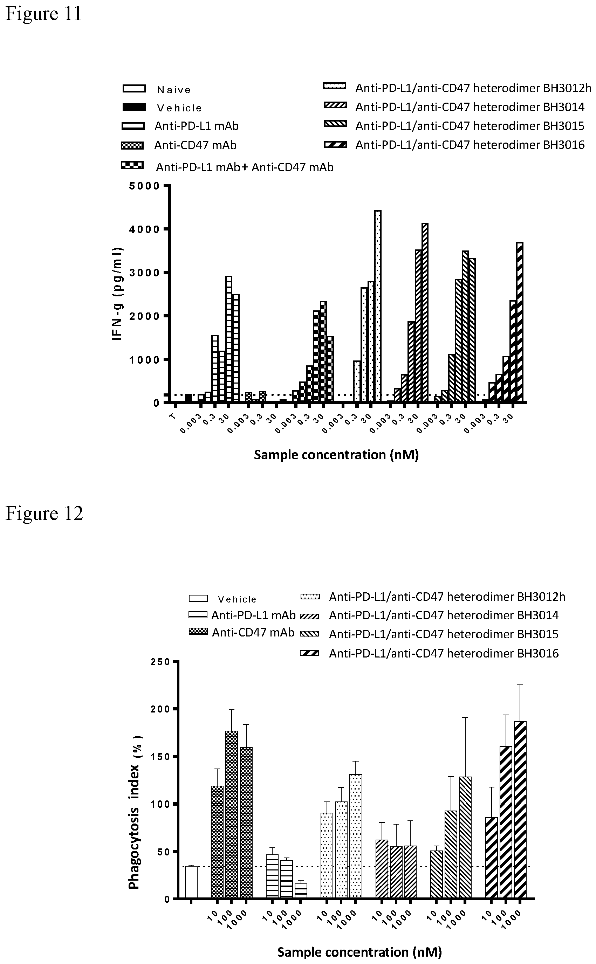

As shown in Figure 11, the human T cells can secrete IFN-γ under the stimulation of allogeneic DCs. The addition of the anti-PD-L1 antibody can enhance the T cell activation and promote the secretion of cytokines. The anti-PD-L1/anti-CD47 heterodimeric antibody also showed very strong T cell modulation activity and significantly promoted the secretion of the cytokine IFN-γ.

### Example 9. Phagocytosis of tumor cells by macrophages mediated by the anti-PD-L1/anti-CD47 heterodimeric antibody

Obtaining of the mature human macrophages: The human PBMC cells (Lonza, Catalog number CC-2702) were thawed and collected, re-suspended in serum-free RPMI 1640 medium at a cell density of 5×10⁶/mL and seeded in a cell culture flask, and cultured in a 37 °C CO₂ incubator for 90 minutes. Discarding the culture supernatant and suspension cells, and the adherent cells were incubated for 7 days in a complete medium (RPMI 1640 with 10% FBS) with the addition of 25ng/ml M-CSF (Sino Biological Inc., catalog number 11792-HNAN). Then the macrophages were harvested and re-suspended in a complete medium (RPMI 1640 with 10% FBS) containing 25ng/ml M-CSF and 50ng/ml IFN- γ (Sino Biological Inc., catalog number 11725-HNAS). This cell suspension was inoculated into a 48- well cell culture plate, with 50,000 cells per well, incubated for 1 day to let the macrophages mature for later use.

Raji tumor cells were stained according to the instruction of the CFSE kit (Life technology, catalog number C34554). Briefly, CFSE was diluted with PBS to a working concentration of 5 µM and prewarmed at 37 °C, centrifuged at 1000rpm for 5 minutes to collect Raji cells, Raji cells were resuspended with prewarmed CFSE working solution, and incubated at 37 °C for 15 minutes. The Raji cells were washed once with the complete medium, resuspend in the complete medium, incubated for 30 minutes, washed twice with the complete medium, and resuspended in the complete medium for later use.

The 48-well plate was washed 3 times with the complete medium. The CFSE-stained Raji cells were pre-incubated with the heterodimer samples to be tested and the control for 15 minutes, then added to the 48-well culture plate, and incubated in a 37 °C CO₂ incubator for 2 hours. After the incubation, the 48-well plate was washed 3 times with the complete medium, and wheat germ agglutinin, alexa fluor 555 (Life technologies, catalog number W32464) diluted in the complete medium was added, 10 µg/ml, and incubated in the dark for 15 minutes. The 48-well plate was further washed 3 times with the complete medium, and fixed with 4 % paraformaldehyde for 15 minutes. The 48-well plate was further washed 3 times with the complete medium, and the complete medium was added therein. Photos were taken under a fluorescence microscope, and the number of cells was counted. The calculation method of the phagocytosis index (%) is: the number of phagocytosed Raji cells labeled with green/the number of the existing macrophages labeled with red × 100.

As shown in Figure 12, the anti-CD47 monoclonal antibody (comprising the heavy and light chain variable region sequences of the humanized sequences z11 of the antibody CD47-18-5 and the constant region sequence of human IgG4) can mediate phagocytosis of Raji tumor cells by macrophages, and all the anti-PD-L1/anti -CD47 heterodimeric antibodies BH3012h, BH3015, and BH3016 can also mediate phagocytosis of Raji tumor cells by macrophages.

### Example 10. Efficacy study of the anti-PD-L1/anti-CD47 heterodimeric antibody against A375 tumor in humanized mice

The 6-8-week-old female immunodeficient NCG mice (Nanjing Biomedical Research Institute) were selected as the experimental material. After the mice acclimated to the environment for one week, the immune system of the mice was partially humanized by intravenously injecting human PBMCs firstly, 5×10⁶ cells/mouse. Each mouse was subcutaneously inoculated with 5×10⁶ A375 human melanoma cells on the right back. Once the tumors grow to a volume of about 100 mm³, the mice were divided into different groups according to the tumor volumes, 6 tumor-bearing mice per group. The vehicle (PBS), the anti-PD-L1 monoclonal antibody 35 nmol/kg, the anti-CD47 monoclonal antibody 35 nmol/kg and the anti-PD-L1/antiCD47 heterodimeric antibody 70 nmol /kg (allowing for the fact that a monoclonal antibody is a bivalent antibody, but the anti-PD-L1 and anti-CD47 portions of the bispecific antibody are both monovalent) were administered respectively, twice a week for 2 consecutive weeks, via an intraperitoneal injection. The tumor volumes were measured 3 times a week beginning at the day of administration, and the long diameter a and the short diameter b were measured and the tumor volume calculation formula is: tumor volume (mm3) = (a × b²)/2. The tumor volume measurement lasted for 3 weeks, i.e., the observation was continued for another 1 week after the medication has been stopped.

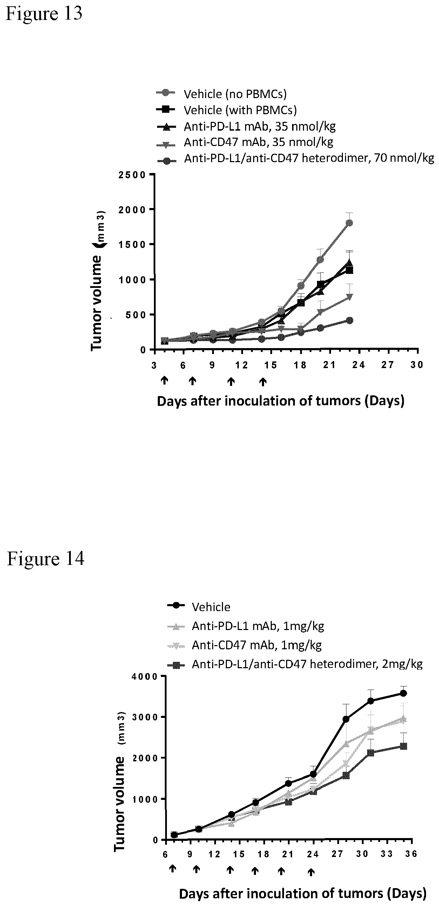

As shown by the results of Figure 13, the anti-PD-L1/anti-CD47 heterodimeric antibodies have a stronger anti-tumor efficacy than the anti-PD-L1 monoclonal antibody and the anti-CD47 monoclonal antibody in the xenograft tumor model.

### Example 11. Efficacy study of the anti-PD-L1/anti-CD47 heterodimeric antibody against MC38 tumor in gene knock-in mice

The 6-8-week-old female B-hPD-L1/hSIRPα/hCD47 triple knock-in mice (Biocytogen Jiangsu Gene Biotechnology Co., Ltd) were selected as the experimental material. After the mice acclimated to the environment for one week, each mouse was subcutaneously inoculated with 5×10⁵ B-hPD-L1/hCD47 MC38 mouse colon tumor cells (in which the mouse-derived PD-L1 and CD47 were knocked out, and the human-derived PD-L1 and CD47 were overexpressed. MC38 cells were purchased from Shunran (Shanghai) Biotechnology Co., Ltd) on the right back. Once the tumors grow to a volume of about 100 mm³, the mice were divided into different groups according to the tumor volumes, 6 tumor-bearing mice per group. The vehicle (PBS), the anti-PD-L1 monoclonal antibody 1 mg/kg, the anti-CD47 monoclonal antibody 1 mg/kg, and the anti-PD-L1/antiCD47 heterodimeric antibody 2 mg/kg (allowing for the fact that a monoclonal antibody is a bivalent antibody, but the anti-PD-L1 and anti-CD47 portions of the bispecific antibody are both monovalent), were administered respectively, twice every week for 3 consecutive weeks, via an intraperitoneal injection. The tumor volumes were measured twice a week beginning at the day of administration, and the long diameter a and the short diameter b were measured and the tumor volume calculation formula is: tumor volume (mm3) = (a × b²)/2. The tumor volume measurement lasted for 3 weeks, i.e., the observation was continued for another 1 week after the medication has been stopped.

As shown by the results of Figure 14, the anti-PD-L1/anti-CD47 heterodimeric antibodies have a stronger anti-tumor efficacy than the anti-PD-L1 monoclonal antibody and the anti-CD47 monoclonal antibody in the homologous tumor model.

### Example 12. Efficacy study of the anti-PD-L1/anti-CD47 heterodimeric antibody against CT26 tumor in wild-type mice

The 6-8-week-old female wild-type Balb/c mice (Beijing Vital River) were selected as the experimental material. After the mice acclimated to the environment for one week, each mouse was subcutaneously inoculated with 5×10⁵ CT26/hPD-L1/hCD47 mouse colon tumor cells (Gempharmatech Co., Ltd, Jiangsu) on the right back. Once the tumors grow to a volume of about 100 mm³, the mice were divided into different groups according to the tumor volumes, 8 tumor-bearing mice per group. The vehicle (DPBS, GIBCO, catalog number 14190-136), the anti-PD-L1 monoclonal antibody 17.5 nmol/kg (the monoclonal antibody corresponding to the anti-PD-L1 portion of the anti-PD-L1/anti-CD47 heterodimeric antibody), the anti-CD47 monoclonal antibody 17.5 nmol/kg (the monoclonal antibody corresponding to the anti-CD47 portion of the anti-PD-L1/anti-CD47 heterodimeric antibody), and the anti-PD-L1/antiCD47 heterodimeric antibody 35 nmol/kg (allowing for the fact that a monoclonal antibody is a bivalent antibody, but the anti-PD-L1 and anti-CD47 portions of the bispecific antibody are both monovalent), were administered respectively, twice every week for 3 consecutive weeks, via an intraperitoneal injection. The tumor volumes were measured twice a week from the day of administration to the end of administration, and once after the end of administration. The long diameter a and the short diameter b were measured each time and the tumor volume calculation formula is: tumor volume (mm3) = (a × b²)/2. The tumor volume measurement lasted for 4 weeks, i.e., the observation was continued for another 1 week after the medication has been stopped.

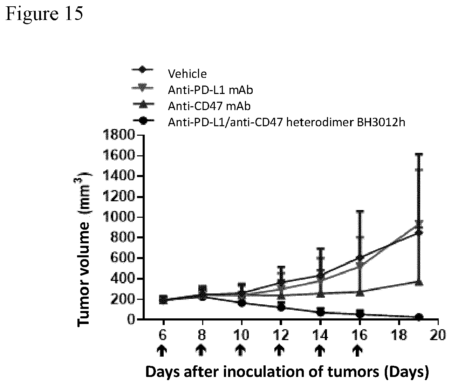

As shown by the results of Figure 15, the anti-PD-L1/anti-CD47 heterodimeric antibody BH3012h has a stronger anti-tumor efficacy than the corresponding anti-PD-L1 bivalent monoclonal antibody and the anti-CD47 bivalent monoclonal antibody in the homologous tumor model.

## Claims

1. A bispecific antibody comprising a first antigen-binding functional region that specifically binds to PD-L1 and a second antigen-binding functional region that specifically binds to CD47, wherein
the first antigen-binding functional region that specifically binds PD-L1 comprises:
(A) a heavy chain variable region, comprising
(a) HCDR1, which comprises the amino acid sequence set forth in SEQ ID NO: 37,
(b) HCDR2, which comprises the amino acid sequence set forth in SEQ ID NO: 38, and
(c) HCDR3, which comprises the amino acid sequence set forth in SEQ ID NO: 39; and
(B) a light chain variable region, comprising
(a) LCDR1, which comprises the amino acid sequence set forth in SEQ ID NO: 40,
(b) LCDR2, which comprises the amino acid sequence set forth in SEQ ID NO: 41, and
(c) LCDR3, which comprises the amino acid sequence set forth in SEQ ID NO: 42.

2. The bispecific antibody of claim 1, wherein
the second antigen-binding functional region that specifically binds to CD47 comprises
(A) a heavy chain variable region, comprising
(a) HCDR1, which comprises the amino acid sequence set forth in SEQ ID NO: 43,
(b) HCDR2, which comprises the amino acid sequence set forth in SEQ ID NO: 44, and
(c) HCDR3, which comprises the amino acid sequence set forth in SEQ ID NO: 45; and
(B) a light chain variable region, comprising
(a) LCDR1, which comprises the amino acid sequence set forth in SEQ ID NO: 46,
(b) LCDR2, which comprises the amino acid sequence set forth in SEQ ID NO: 47, and
(c) LCDR3, which comprises the amino acid sequence set forth in SEQ ID NO: 48.

3. The bispecific antibody of claim 1 or claim 2, wherein
the first antigen-binding functional region that specifically binds to PD-L1 comprises
(A) a heavy chain variable region, comprising
(a) HCDR1, which comprises the amino acid sequence set forth in SEQ ID NO: 37,
(b) HCDR2, which comprises the amino acid sequence set forth in SEQ ID NO: 38, and
(c) HCDR3, which comprises the amino acid sequence set forth in SEQ ID NO: 39; and
(B) a light chain variable region, comprising
(a) LCDR1, which comprises the amino acid sequence set forth in SEQ ID NO: 40,
(b) LCDR2, which comprises the amino acid sequence set forth in SEQ ID NO: 41, and
(c) LCDR3, which comprises the amino acid sequence set forth in SEQ ID NO: 42; and
the second antigen-binding functional region that specifically binds to CD47 comprises
(A) a heavy chain variable region, comprising
(a) HCDR1, which comprises the amino acid sequence set forth in SEQ ID NO: 43,
(b) HCDR2, which comprises the amino acid sequence set forth in SEQ ID NO: 44, and
(c) HCDR3, which comprises the amino acid sequence set forth in SEQ ID NO: 45; and
(B) a light chain variable region, comprising
(a) LCDR1, which comprises the amino acid sequence set forth in SEQ ID NO: 46,
(b) LCDR2, which comprises the amino acid sequence set forth in SEQ ID NO: 47, and
(c) LCDR3, which comprises the amino acid sequence set forth in SEQ ID NO: 48.

4. The bispecific antibody of any one of claims 1-3, wherein the first antigen-binding functional region that specifically binds to PD-L1 comprises a heavy chain variable region comprising the amino acid sequence set forth in SEQ ID NO: 6; and a light chain variable region comprising the amino acid sequence set forth in SEQ ID NO: 2.

5. The bispecific antibody of any one of claims 1-4, wherein the second antigen-binding functional region that specifically binds to CD47 comprises a heavy chain variable region comprising the amino acid sequence set forth in SEQ ID Nos: 14, 20, 24, 28, 32 or 36; and a light chain variable region comprising the amino acid sequence set forth in SEQ ID Nos: 12, 18, 22, 26, 30 or 34.

6. The bispecific antibody of any one of claims 1-5,
wherein the first antigen-binding functional region that specifically binds to PD-L1 comprises a heavy chain variable region comprising the amino acid sequence set forth in SEQ ID NO: 6; and a light chain variable region comprising the amino acid sequence set forth in SEQ ID NO: 2; and
wherein the second antigen-binding functional region that specifically binds to CD47 comprises a heavy chain variable region and a light chain variable region selected from the following combinations:
a) the heavy chain variable region comprising the amino acid sequence set forth in SEQ ID NO: 14, and the light chain variable region comprising the amino acid sequence set forth in SEQ ID NO: 12;
b) the heavy chain variable region comprising the amino acid sequence set forth in SEQ ID NO: 20, and the light chain variable region comprising the amino acid sequence set forth in SEQ ID NO: 18;
c) the heavy chain variable region comprising the amino acid sequence set forth in SEQ ID NO: 24, and the light chain variable region comprising the amino acid sequence set forth in SEQ ID NO: 22;
d) the heavy chain variable region comprising the amino acid sequence set forth in SEQ ID NO: 28, and the light chain variable region comprising the amino acid sequence set forth in SEQ ID NO: 26;
e) the heavy chain variable region comprising the amino acid sequence set forth in SEQ ID NO: 32, and the light chain variable region comprising the amino acid sequence set forth in SEQ ID NO: 30;
f) the heavy chain variable region comprising the amino acid sequence set forth in SEQ ID NO: 36, and the light chain variable region comprising the amino acid sequence set forth in SEQ ID NO: 34.

7. The bispecific antibody of any one of claims 1-6, wherein the first antigen-binding functional region and the second antigen-binding functional region are selected from the group consisting of Fab fragments, scFv fragments, and variable domain fragments Fv.

8. The bispecific antibody of any one of claims 1-7, wherein the first antigen-binding functional region and the second antigen-binding functional region are both Fab fragments.

9. The bispecific antibody of claim 8, wherein the Fab fragment thereof comprises a different first heavy chain variable region and a different second heavy chain variable region, as well as a different first light chain variable region and a different second light chain variable region.

10. The bispecific antibody of any one of claims 1-7, wherein one of the first antigen-binding functional region and the second antigen-binding functional region is a Fab fragment, and the other is a scFv.

11. The bispecific antibody of any one of claims 1-7, comprising a first Fc chain and a second Fc chain,
wherein the first Fc chain and the second Fc chain are both immunoglobulin G Fc fragments containing amino acid substitutions, and the first Fc chain and the second Fc chain together constitute a heterodimer that can bind to the Fc receptor;
wherein the first Fc chain and the second Fc chain are linked to the first antigen-binding functional region and the second antigen-binding functional region, respectively, via a covalent bond or a linker; and
wherein either the first Fc chain or the second Fc chain comprises T366L and D399R amino acid substitutions at positions 366 and 399, and the other comprises L351E, Y407L and K409V amino acid substitutions at positions 351, 407 and 409, wherein the amino acid positions are numbered according to the Kabat EU index numbering system.

12. The bispecific antibody of claim 11, wherein the weight ratios of the homodimers formed by the first Fc chain and the first antigen-binding functional region covalently linked thereto and by the second Fc chain and the second antigen -binding functional region covalently linked thereto of said bispecific antibody are less than 50% of the total amount of all the polypeptide chains, in a reducing agent-containing solution in which no other polypeptides present except for said Fc chains and the antigen-binding functional regions.

13. The bispecific antibody of any one of claims 1-12, comprising
a first heavy chain and first light chain pair that specifically binds to PD-L1, wherein the first heavy chain has a heavy chain variable region comprising the amino acid sequence set forth in SEQ ID NO: 6, and a heavy chain constant region comprising the amino acid sequence set forth in SEQ ID NO: 8 or 10; the first light chain has a light chain variable region comprising the amino acid sequence set forth in SEQ ID NO: 2, and a light chain constant region comprising the amino acid sequence set forth in SEQ ID NO: 4.

14. The bispecific antibody of any one of claims 1-13, comprising
a second heavy chain and second light chain pair that specifically binds to CD47, wherein the second heavy chain has a heavy chain variable region comprising the amino acid sequence set forth in SEQ ID Nos: 14, 20, 24, 28, 32 or 36, and a heavy chain constant region comprising the amino acid sequence set forth in SEQ ID NO: 16; the second light chain has a light chain variable region comprising the amino acid sequence set forth in SEQ ID Nos: 12, 18, 22, 26, 30 or 34, and a light chain constant region comprising the amino acid sequence set forth in SEQ ID NO: 4.

15. An isolated polynucleotide encoding the bispecific antibody of any one of claims 1-14.

16. A recombinant expression vector comprising the isolated polynucleotide of claim 15.

17. A host cell comprising the isolated polynucleotide of claim 15 or the recombinant expression vector of claim 16.

18. The host cell of claim 17, selected from the group consisting of human embryonic kidney cells HEK293, or HEK293T, HEK293E, HEK293F that are modified from HEK293 cells; hamster ovary cells CHO, or CHO-S, CHO-dhfr-, CHO/DG44, ExpiCHO that are modified from CHO cells; *Escherichia coli,* or *Escherichia coli* BL21, BL21(DE3), Rosetta, Origami that are modified from *E. coli;* Yeast, or *Pichia, Saccharomyces cerevisiae, Kluyveromyces lactis, Hansenula polymorpha* that are modified from Yeast; insect cells, or High5, SF9 cells that are modified from insect cells; plant cells; mammalian mammary gland cells; somatic cells.

19. A composition comprising the bispecific antibody of any one of claims 1-14 or the isolated polynucleotide of claim 15 or the recombinant expression vector of claim 16 or the host cell of claim 17 or 18, and a pharmaceutically acceptable carrier.

20. A method of producing the bispecific antibody of any one of claims 1-14, including the steps of:
1) Expressing the isolated polynucleotide of claim 15 or the recombinant expression vector of claim 16 in host cells, respectively;
2) Reducing the proteins respectively expressed in the host cells; and
3) Mixing the reduced proteins, and then oxidizing the mixture.

21. The method of claim 19 or 20, wherein the reduction step includes 1) performing the reduction reaction in the presence of a reducing agent selected from the group consisting of 2-mercaptoethylamine, dithiothreitol, tris(2-carboxyethyl) phosphine or other chemical derivatives thereof; 2) removing the reducing agent.

22. The method of any one of claims 19-21, wherein the oxidation step is conducted in the air or in the presence of an oxidizing agent selected from the group consisting of L-dehydroascorbic acid or chemical derivatives thereof.

23. The method of any one of claims 19-22, further including a step of separation and purification.

24. Use of the bispecific antibody of any one of claims 1-14 and/or the isolated polynucleotide of claim 15 and/or the recombinant expression vector of claim 16 and/or the host cell of claim 17 or 18 and/or the composition of claim 19, in the manufacture of a medicament for preventing and/or treating diseases in a subject.

25. The bispecific antibody of any one of claims 1-14 and/or the isolated polynucleotide of claim 15 and/or the recombinant expression vector of claim 16 and/or the host cell of claim 17 or 18 and/or the composition of claim 19, for use as a medicament for preventing and/or treating diseases in a subject.

26. A method of preventing and/or treating diseases, including administering to a subject in need thereof the bispecific antibody of any one of claims 1-14 and/or the isolated bispecific antibody of claim 15 and/or the recombinant expression vector of claim 16 and/or the host cell of claim 17 or 18 and/or the composition of claim 19.

27. Use of claim 24, the bispecific antibody in a heterodimeric form, the isolated polynucleotide, the recombinant expression vector, the host cell, or the composition of claim 25, or the method of claim 26, wherein the subject is a mammal, preferably a human subject.

28. Use of claim 24, the bispecific antibody in a heterodimeric form, the isolated polynucleotide, the recombinant expression vector, the host cell or the composition of claim 25, or the method of claim 26, wherein the diseases are selected from the group consisting of the following tumors: leukemia, lymphoma, myeloma, brain tumor, head and neck squamous cell cancer, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell cancer, melanoma, small cell lung cancer and bone cancer.
